(19) Europäisches Patentamt
European Patent Office
Office européen des brevets

(11) EP 4 481 105 A1

(12) EUROPEAN PATENT APPLICATION

(43) Date of publication:
**25.12.2024 Bulletin 2024/52**

(21) Application number: **23305984.9**

(22) Date of filing: **21.06.2023**

(51) International Patent Classification (IPC):
***D06M 11/05*** (2006.01) ***D01F 1/10*** (2006.01)
***D01F 6/14*** (2006.01) ***D01F 6/50*** (2006.01)
***A61L 27/52*** (2006.01)

(52) Cooperative Patent Classification (CPC):
(C-Sets available)
**D06M 11/05; A61L 15/12; A61L 15/14; A61L 27/16; A61L 27/50; A61L 27/52; A61L 31/048; A61L 31/14; A61L 31/145; D01F 6/14;**
A61L 2430/10; A61L 2430/34 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Ecole Nationale Superieure des Mines de Paris 75272 Paris cedex 06 (FR)**
- **Association pour la Recherche et le Développement des Méthodes et Processus Industriels (ARMINES) 75272 Paris Cedex 06 (FR)**

(72) Inventors:
- **CORTE, Laurent 94400 Vitry-sur-Seine (FR)**
- **CAROUX, Julien 91300 Massy (FR)**

(74) Representative: **Icosa 83 avenue Denfert-Rochereau 75014 Paris (FR)**

# (54) FATIGUE RESISTANT SWOLLEN FIBER ASSEMBLIES

(57) The present invention relates a swollen fiber assembly comprising at least two swollen fibers, each swollen fiber comprising a polymer and at least one swelling agent, the polymer being swollen with the least one swelling agent and consisting of at least two polymer chains, the at least two polymer chains being semi-crystalline, cross-linked together by weak bonds in amorphous areas, and oriented on a macromolecular scale in the swollen state, the at least two fibers being non-woven, twisted, woven, braided or knitted together, or included within a polymeric matrix further comprised in the swollen fiber assembly. The present invention also relates to a method for obtaining a self-recovery phenomenon of said swollen fiber assembly.



Processed by Luminess, 75001 PARIS (FR)

(Cont. next page)

(52) Cooperative Patent Classification (CPC): (Cont.)

C-Sets
**A61L 15/12, C08L 29/04;**
**A61L 27/16, C08L 29/04;**
**A61L 31/048, C08L 29/04**

## Description

## FIELD OF INVENTION

The present invention relates to swollen fibers and their assemblies that are fatigue resistant, i.e. after a mechanical stress, they recover all or part of their mechanical behavior (dimension, stiffness, hysteresis) by simply applying a resting time.

## BACKGROUND OF INVENTION

Synthetic hydrogels are of utmost interest for the reconstruction of soft biological tissues. As compared to other classes of synthetic materials, they can mimic closely the physics and physical-chemistry of biological tissues. As compared to biosourced systems, they offer the versatility, safety, availability and reproducibility of synthetic polymer chemistry. Chemical functions and modifications have now been proven to show various levels of biocompatibility from moderate biotolerance to bioactivity. Nevertheless, insufficient resistance to tensile loadings remains a major limitation for the reconstruction of soft tissues. Making hydrogels with high tensile performances, strength, stiffness and fatigue resistance, is a major unmet challenge. Strategies proposed so far to enhance these performances require to find a compromise as they mostly imply the introduction of fiber fillers, therefore raising problems of matrix-fiber cohesion and biocompatibility of the fiber component.

It is essential that the materials used for soft tissue reconstruction or reinforcement are able to sustain repeated mechanical loadings representative of their conditions of use. This fatigue resistance not only consists in avoiding the rupture but also in maintaining the functionality in terms of geometry and mechanical response. For instance, in the case of ligament reconstruction, soft tissue substitutes should not creep to a point where they induce a laxity of the reconstructed joint. Most of the works that have been reported recently on the fatigue resistance of hydrogels focus on the resistance to rupture.

In addition, in the field of robotics, there is also a need to have soft structural elements that have a durable mechanical function, especially in the joints of the robot or in the soft robotic systems where the motion of stiff articulated rods is replaced by the deformation of soft materials. Several soft robots have been reported for applications in aqueous environment such as in vivo or undersea applications. Hydrogels are particularly appropriate materials in these contexts. Here also, the resistance to fatigue including the stability of the geometry and mechanical behavior under cyclic loadings is essential to ensure the precision and reproducibility of the motion.

Therefore, the present invention aims at providing a swollen fiber assembly that can be used in the fields of mammalian surgery and/or orthopedics and robotics and that has high tensile properties and enhanced fatigue resistance.

## SUMMARY

The present invention refers to a swollen fiber assembly comprising at least two swollen fibers, each swollen fiber comprising a polymer and at least one swelling agent, the polymer being swollen with the least one swelling agent and consisting of at least two polymer chains, the at least two polymer chains being: semi-crystalline, cross-linked together by weak bonds in amorphous areas, and oriented on a macromolecular scale in the swollen state; the at least two fibers being non-woven, twisted, woven, braided or knitted together, or included within a polymeric matrix further comprised in the swollen fiber assembly.

Advantageously, the at least two polymer chains are linked together by hydrogen bonds and/or the amorphous areas are located between crystallites in the direction of the length of each chain of the at least one polymer.

Advantageously, the crystallinity of each fiber is greater than 15%, preferably it ranges from 20% to 80%, more preferably it ranges from 25% to 70%, even more preferably it ranges from 30% to 65%, the crystallinity being measured by differential scanning calorimetry on the fibers in the dry state.

Advantageously, the weight of the polymer of each swollen fiber represents from 5% to 90% of the total weight of said swollen fiber at room temperature, preferably the weight of the polymer of each swollen fiber represents from 20% to 80% of the total weight of said swollen fiber at room temperature, more preferably the weight of the polymer of each swollen fiber represents from 30% to 70% of the total weight of said swollen fiber at room temperature, even more preferably the weight of the polymer of each swollen fiber represents about 50% of the total weight of said swollen fiber at room temperature. Preferably, the room temperature is 20°C.

Advantageously, the polymer is selected from the group consisting of: poly(vinyl alcohol)s, poly(urethane)s, poly(ester)s, poly(saccharide)s, proteins, synthetic polypeptides, and any copolymers thereof, preferably the polymer consists of poly(vinyl alcohol).

Advantageously, the at least two polymer chains are poly(vinyl alcohol) chains, preferably chains of poly(vinyl alcohol) having an average molar mass ranging from 5,000 g/mol to 400,000 g/mol, more preferably from 25,000 g/mol to

300,000 g/mol, even more preferably from 80,000 g/mol to 200,000 g/mol.

Advantageously, the linear density of each of the at least two swollen fibers is greater than 0.1 Dtex, preferably greater than 1 Dtex, more preferably greater than 10 Dtex, even more preferably greater than 100 Dtex.

Advantageously, the diameter of each swollen fiber ranges from 1 μm to 1000 μm, preferably from 5 μm to 500 μm, more preferably from 10 μm to 200 μm, even more preferably from 15 μm to 150 μm.

Advantageously, the at least one swelling agent is selected from the group consisting of water, dimethyl sulfoxide, dimethylformamide and mixtures thereof, preferably the at least one swelling agent is water.

Advantageously, the weight of the at least one swelling agent represents at least 10% of the total weight of each swollen fiber at room temperature, preferably the weight of the at least one swelling agent represents from 20% to 80% of the total weight of each swollen fiber at room temperature, more preferably the weight of the at least one swelling agent represents from 30% to 70% of the total weight of each swollen fiber at room temperature, even more preferably the weight of the at least one swelling agent represents about 50% of the total weight of each swollen fiber at room temperature. Preferably, the room temperature is 20°C.

The present invention also relates to a method for obtaining a self-recovery phenomenon of a swollen fiber assembly according to the invention, comprising the following steps: applying a constant mechanical stress or a loading-unloading cycle or a series of loading-unloading cycles on said swollen fiber assembly, then applying a resting time of at least 30 minutes, preferably of at least 1 hour, more preferably of at least 2 hours, even more preferably of about 8 hours, on the swollen fiber assembly.

The present invention further relates to the use of a swollen fiber assembly according to the invention as or in an implant, as or in an orthosis, as or in exoskeleton, or in a robotic structure.

## DEFINITIONS

In the present invention, the following terms have the following meanings:

"**About**", before a figure or number, refers to plus or minus 10% of the face value of that figure or number. In one embodiment, "about", before a figure or number, refers to plus or minus 5% of the face value of that figure or number.

"**Axis of a fiber**" refers to the axis in the direction of the fiber length.

"**Axis of a yarn**" refers to the axis in the direction of the yarn length.

"**Axis of a structure**" refers to the axis in the direction of the longest dimension of a structure, in particular in the direction of the structure length. For example, the structure may be a fiber assembly having the form of a yarn, a twisted fabric such as a cord, a woven fabric, a non-woven fabric, a braided fabric or a knitted fabric.

"**Comprising**" or "**comprise**" is to be construed in an open, inclusive sense, not limited to the features following this term.

"**Consisting of**" or "**consist**" is to be construed in a close, non-inclusive sense, limited to the features following this term.

"**Crosslink**" refers to a bond or a short sequence of bonds between two polymer chains. The crosslinks are chemical or physical. The chemical crosslinks correspond to covalent bonds between polymer chains. Preferably, the chemical crosslinks are obtained by using at least one of the following crosslinking methods: use of a multifunctional chemical agent (for example multi-aldehydes such as glutaraldehyde or glyoxal, multi-thiols, multi-acrylates, multi-carboxylic-acids, multi-dienes), use of irradiation (for example gamma-ray or electron-beam irradiation) or use of chemical reaction between reactive moieties of the polymer chains. The physical crosslinks correspond to non-covalent bonds between polymer chains (for example hydrogen bonds, crystallites, pi-pi stacking, ionic interactions, hydrophobic bonds). Preferably, the physical crosslinks are obtained by using at least one of the following crosslinking methods: crosslinking by crystallites in the case of polymer chains with crystallizable segments using solvent casting, freezing/thawing, liquid-liquid phase separation and heat treatment; crosslinking by ionic complexation (for example with borax); and crosslinking by mesophase separation in the case of copolymer chains.

"**Crystallite**" refers to a domain of a polymer having the same structure as a single crystal, that is a domain in which the atoms composing the polymer chains are arranged regularly onto a crystal lattice that extends in all directions. Within a crystallite, polymer chains are perfectly oriented parallel to each other along one crystalline axis. In one embodiment, the crystallites are insoluble in a swelling agent at the temperature of use. A "**semi-crystalline polymer**" refers to a polymer comprising crystallites and amorphous regions of said polymer, wherein the crystallites of said polymer are connected to each other by the amorphous regions of said polymer. The "**crystallinity**" of a semi-crystalline polymer refers to the fraction of polymer mass or the fraction of polymer segments that compose the crystallites over the total polymer mass or total number of polymer segments, respectively.

"**Dtex**" refers to the weight in grams of 10,000 meters of fiber.

"**Dry state**" refers to the state in the absence of swelling agent impregnation of a material, comprising less than 10% by weight of any swelling agent, preferably comprising less than 5% by weight of any swelling agent.

"**Fatigue**" refers to a weakening of a material under cyclic loading, which can lead to creep, stiffness reduction,

localized damage or even failure of said material even though the stress is below the ultimate stress or yield stress limit.

"**Fiber**" refers to a thin, threadlike structure made of at least two polymer chains cross-linked between each other. According to the invention, a fiber in a dry state has a length ranging from 1 mm to 10 km, preferably ranging from 1 mm to 10 m, more preferably ranging from 1 mm to 1 m. According to the invention, a fiber in a dry state has a diameter ranging from 0.01 μm to 1000 μm, preferably ranging from 0.1 μm to 500 μm, more preferably ranging from 1 μm to 200 μm, even more preferably ranging from 10 μm to 150 μm.

"**From X to Y**" refers to the range of values between X and Y, the limits X and Y being included in said range.

"**Gel**" refers to a solid three-dimensional network of cross-linked polymer chains, percolating through a given volume. The gel can be swollen but not dissolved by a liquid (the liquid is referred to as "**swelling agent**") and exhibits the properties of a solid ranging from ductile to brittle. A chemical gel refers to a gel where the crosslinks are chemicals. A physical gel refers to a gel where the crosslinks are physicals. In a physical gel where crosslinks are crystallites, the crystallinity is typically greater than 5%, preferably the crystallinity ranges from 10% to 80%, more preferably from 20% to 65%, the crystallinity being measured by differential scanning calorimetry.

"**Hydrogel**" refers to a gel in which the swelling agent is water.

"**Hydrogel fibers**" refers to fibers made of a hydrogel, that is fibers made of a gel comprising a cross-linked polymer matrix and water as the gel swelling agent.

"**Hydrolysis degree of PVA**" refers to the percentage of the number of vinyl alcohol monomers relative to the total number of monomers present in the PVA. Indeed, PVA is obtained from hydrolysis of poly(vinyl acetate) (PVAc) but this hydrolysis process is not perfect and there are always some remaining acetate functions along the chain; the hydrolysis degree of PVA gives the percentage of vinyl alcohol monomers.

"**Implant**" refers to an exogenous device inserted into the body of a mammal. "**Prosthesis**" or "**tissue substitutes**" refer to implants which are used to repair or replace an element of the body of a mammal, for example a soft biological tissue. In one embodiment, "implant" or "prosthesis" or "tissue substitutes" refers to an exogenous device inserted into the body of a human to repair or replace an element of said human body, for example a soft biological tissue such as a ligament, for example the anterior cruciate ligament (ACL) or an artery such as the aorta. In one embodiment, "implant" or "prosthesis" or "tissue substitutes" refers to an exogenous device inserted into the body of a human to repair or replace a biological tissue. In one embodiment, "implant" or "prosthesis" or "synthetic substitutes" refers to an exogenous device inserted into the body of a non-human mammal, such as horse or a dog, to repair or replace an element of said body, for example a soft biological tissue such as a ligament, for example the cranial cruciate ligament (CrCL).

"**Ligaments**" refers to a ligament of a mammal. In one embodiment, the ligament is selected from the group consisting of head and neck ligaments (cricothyroid ligament, periodontal ligament, suspensory ligament of the lens), wrist ligaments (palmar radiocarpal ligament, dorsal radiocarpal ligament, ulnar collateral ligament, radial collateral ligament), shoulder ligament (rotator cuff), and knee ligaments (anterior cruciate ligament (ACL), lateral collateral ligament (LCL), posterior cruciate ligament (PCL), medial collateral ligament (MCL), cranial cruciate ligament (CrCL) - quadruped equivalent of ACL, caudal cruciate ligament (CaCL) - quadruped equivalent of PCL, patellar ligament).

"**Oriented on a macromolecular scale in the swollen state**" refers to a polymer material in which segments of the backbone of polymer chains are, in the swollen state, mostly oriented in one same direction within a macroscopic volume of said material. In one embodiment, the swollen state oriented on a macromolecular scale is obtained from the swelling of a polymer material in which segments of the backbone of polymer chains are, in the dry state, mostly oriented in one same direction within a macroscopic volume of said material; the dry state oriented on a macromolecular scale may be obtained, for example, by stretching a polymer material in the dry state in one direction above the glass transition temperature $T_g$ or the melting temperature $T_m$ of the oriented polymer segments and then cooling it below $T_g$ or $T_m$ while maintaining the stretching. A fiber according to the invention is oriented on a macromolecular scale in the swollen state, i.e. the polymer chains composing the swollen fiber have segments of their backbone mostly oriented along the fiber axis. In one embodiment, the term "mostly oriented in one same direction within a macroscopic volume of said material" means that the Hermans orientation factor is strictly greater than zero (Hermans orientation factor > 0). Advantageously, the Hermans orientation factor ranges from 0.1 to 1.0, more advantageously from 0.3 to 1.0.

"**Pitch**" or "**lay length**" refers to the length of a yarn (or twisted structure made of yarns) on which a fiber (or yarn) makes a complete turn. It is equal to the inverse of the twisting rate.

"**Poly**(**vinyl alcohol**)" or "**PVA**" is a polymer with a hydrocarbon backbone and pendant hydroxyl groups, said polymer having the following structure:

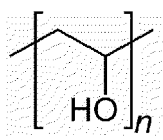,

wherein n is typically ranging from 100 to 10,000.

In one embodiment, PVA is used as the at least one polymer according to the invention; in this embodiment, the PVA chains have:

- a number of monomers ranging from 100 to 10,000, preferably from 500 to 7,000;
- a mass average molar mass (Mw) ranging from 5,000 to 400,000 g/mol, preferably from 25,000 to 300,000 g/mol, even more preferably from 80,000 to 200,000 g/mol;
- a glass transition temperature ($T_g$) ranging from 50°C to 95°C, preferably from 60°C to 89°C;
- a melting point ranging from 200°C to 270°C, preferably from 210°C to 250°C;
- and/or a hydrolysis degree ranging from 70% to 100%, preferably from 80% to 99.99%.

"**Polymer**" refers to a material comprising at least two polymer chains.

"**Polymer chain**" refers to a linear or branched polymer chain, the backbone of which typically comprises more than 10 monomers linked covalently in series, preferably more than 100 monomers.

"**Polymer segment**" means a portion of at least 2 consecutive covalent bonds of the backbone of a chain of that polymer.

"**Resting time**" refers to a time during which no mechanical stress is applied to a structure, for example to a swollen fiber assembly.

"**Room temperature**" refers to a temperature ranging from 20°C to 25°C. Unless specified otherwise, a specific "room temperature" refers to a temperature of 20°C.

"**Soft biological tissues**" or "**soft tissues**" refers to the non-mineralized tissues of a mammal, for example a tissue selected from the group consisting of: fatty tissues, muscles, tendons, ligaments, fasciae, skin, blood vessels, lymphatic vessels, cartilage, menisci and nerves. In an embodiment, "soft biological tissues" or "soft tissues" refers to tendons and ligaments. In a preferred embodiment, "soft biological tissues" or "soft tissues" refers to a ligament. In a more preferred embodiment, "soft biological tissues" or "soft tissues" refers to anterior cruciate ligament (ACL).

"**Swelling agent**" refers to a fluid, in particular a liquid, used to swell a gel, network or solid. In one embodiment, "swelling agent" refers to a liquid, used to swell a gel, network or solid, said liquid being selected from the group consisting of: water, aqueous solutions and biological fluids.

"**Swollen state**" refers to the state in the presence of swelling agent impregnation of a material, comprising more than 10% by weight of any swelling agent, preferably comprising more than 30% by weight of any swelling agent, more preferably comprising more than 40% by weight of any swelling agent.

"**Twisting rate**" or "**twist**" refers to the number of turns per unit length of fiber (in a yarn) or of yarn (in a twisted structure made of yarns). It is equal to the inverse of the pitch.

"**Weak bonds**" or "**physical bonds**" refers to non-covalent attractive interactions between polymer chains (for example hydrogen bonds, pi-pi stacking, hydrophobic interactions, ionic bonds). In one embodiment, "weak bonds" or "physical bonds" refers to hydrogen bonds.

"**Yarn**" refers to an assembly of at least two swollen fibers according to the invention, the at least two fibers being either twisted or parallel from each other. In one embodiment, a yarn comprises about 15 swollen fibers according to the invention. In another embodiment, a yarn comprises about 100 swollen fibers according to the invention.

## DETAILED DESCRIPTION

### Swollen fiber assembly

This invention relates to a swollen fiber made of a material swollen with at least one swelling agent, the material comprising at least one polymer consisting of at least two polymer chains, the at least two polymer chains being:

- semi-crystalline,
- oriented on a macromolecular scale in the swollen state, and
- linked together by weak bonds such as hydrogen bonds in amorphous areas, preferably the amorphous areas being located between crystallites in the direction of the length of each chain of the at least one polymer.

This invention also relates to a swollen fiber assembly comprising at least two swollen fibers, each swollen fiber being made of a material swollen with at least one swelling agent, the material comprising at least one polymer consisting of at least two polymer chains, the at least two polymer chains being:

- semi-crystalline,
- oriented on a macromolecular scale in the swollen state,

- linked together by weak bonds such as hydrogen bonds in amorphous areas, the amorphous areas being preferably located between crystallites in the direction of the length of each chain of the at least one polymer.

the at least two fibers being non-woven, twisted, woven, braided or knitted together, or included within a polymeric matrix further comprised in the swollen fiber assembly.

The polymer

The backbone of the at least two polymer chains contains at least one segment of at least 10 monomers representing at least 10% of the polymer monomer units. In one embodiment, the at least one segment has a melting temperature $T_m$ in contact with a swelling agent, at least 5°C greater than the temperature of use. Preferably, the temperature of use is room temperature (20°C-25°C, preferably 20°C) or body temperature (37°C).

Advantageously, the polymer of each swollen fiber is selected from the group consisting of: poly(vinyl alcohol)s, poly(urethane)s, poly(ester)s, poly(saccharide)s, proteins, synthetic polypeptides, and any copolymers thereof. More advantageously, the polymer of each swollen fiber is selected from the group consisting of poly(vinyl alcohol)s and any copolymers containing poly(vinyl alcohol).

In one embodiment, the polymer comprises poly(vinyl alcohol) (=PVA). In a preferred embodiment, the at least two polymer chains are made of poly(vinyl alcohol).

Advantageously, the PVA is highly hydrolyzed, in particular the hydrolysis degree of PVA is equal or greater than 90%, more preferably the hydrolysis degree of PVA is greater than 95%, even more preferably the hydrolysis degree of PVA is from 98% to 99.99%. A highly hydrolyzed PVA, in particular a PVA having a hydrolysis degree equal or greater than 90%, advantageously greater than 95%, more advantageously from 98% to 99.99%, is preferred because acetate groups have been reported to be proinflammatory; in addition, they decrease the crystallization of the PVA and increase the solubility of the PVA.

Advantageously, the PVA has a melting point of about 230°C.

Advantageously, the glass transition temperature ($T_g$) of the PVA in its dry state is from 70°C to 90°C, preferably the glass transition temperature ($T_g$) of the PVA is 85°C.

More advantageously, the PVA has a hydrolysis degree from 98% to 99.9%, a melting point of 230°C and a glass transition temperature ($T_g$) of 85°C.

Preferably, the PVA has an average molar mass ranging from 5,000 to 400,000 g/mol, preferably from 25,000 to 300,000 g/mol, even more preferably from 80,000 to 200,000 g/mol.

In one embodiment, the weight of the at least two polymer chains represents from 5% to 90% of the total weight of each swollen fiber at room temperature, preferably the weight of the at least two polymer chains represents from 20% to 80% of the total weight of each swollen fiber at room temperature, more preferably the weight of the at least two polymer chains represents from 30% to 70% of the total weight of each swollen fiber at room temperature, even more preferably the weight of the at least two polymer chains represents about 50% of the total weight of each swollen fiber at room temperature.

PVA gels, in particular PVA hydrogels, have the advantages of being stable, non-resorbable materials and very well tolerated *in vivo*: they are nontoxic and have good biocompatibility characteristics, provided that the hydrolysis degree is equal or greater than 90%.

Crosslinking

The at least two polymer chains of the polymer of each swollen fiber are chemically and/or physically crosslinked with each other.

The crosslinking between the at least two polymer chains signifies that there is at least one crosslink between one polymer chain and at least one other polymer chain. The number of cross-links must be sufficient to obtain a gel.

In one embodiment, the at least two polymer chains are chemically crosslinked with each other. Preferably, the at least two polymer chains are chemically crosslinked with each other by using at least one of the following crosslinking methods: use of chemical agents (for example glutaraldehyde, glyoxal, maleic acid, citric acid, trisodium trimetaphosphate, sodium hexametaphosphate, dianhydrides, succinic acid, and sulfosuccinic acid) and use of irradiation (for example gamma-ray or electron-beam irradiation). More preferably, the at least two polymer chains are chemically crosslinked by using at least one of the following crosslinking methods: use of chemical agents selected from the group consisting of glutaraldehyde and glyoxal, and use of gamma-ray irradiation.

In another embodiment, the at least two polymer chains are physically crosslinked with each other. Preferably, the at least two polymer chains are physically crosslinked with each other using at least one of the following crosslinking methods: crosslinking by crystallites in the case of polymer chains with crystallizable segments using solvent casting, freezing/thawing, liquid-liquid phase separation and heat treatment; crosslinking by ionic complexation (for example with

borax); and crosslinking by mesophase separation in the case of copolymer chains.

In another embodiment, the at least two polymer chains are chemically and physically crosslinked with each other. Preferably, the at least two polymer chains are chemically crosslinked with each other by using at least one of the following crosslinking methods: use of chemical agents (for example glutaraldehyde, glyoxal, maleic acid, citric acid, trisodium trimetaphosphate, sodium hexametaphosphate, dianhydrides, succinic acid, and sulfosuccinic acid) and use of irradiation (for example gamma-ray or electron-beam irradiation); and physically crosslinked with each other by using at least one of the following crosslinking methods: crosslinking by crystallites in the case of polymer chains with crystallizable segments using solvent casting, freezing/thawing, liquid-liquid phase separation and heat treatment; crosslinking by ionic complexation (for example with borax); and crosslinking by mesophase separation in the case of copolymer chains.

In one embodiment, the manufacture of the swollen fiber according to the invention comprises a step of physically and/or chemically crosslinking the at least two polymer chains of said swollen fiber. The person skilled in the art knows how to adapt the crosslinking method and/or the crosslinking conditions depending for instance on the type of the polymer(s) comprised in the fibers and on the desired crosslinking rate.

Advantageously, the crosslinks between the at least two polymer chains correspond to crystallites. More advantageously, the crystallinity of the polymer of each swollen fiber, measured by wide angle X-ray diffraction in the dry state, is greater than 10%, preferably from 15% to 70%, more preferably from 20% to 60%, even more preferably from 25% to 55%.

Advantageously, the crosslinks between the at least two polymer chains correspond to crystallites. More advantageously, the crystallinity of the polymer of each swollen fiber, measured by differential scanning calorimetry in the dry state, is greater than 15%, preferably from 20% to 80%, more preferably from 25% to 70%, even more preferably from 30% to 65%.

<u>Orientation of the polymer chains</u>

The at least two polymer chains of the polymer of each swollen fiber are oriented on a macromolecular scale in the swollen state in said fiber, preferably they are oriented in the direction of the fiber length.

Advantageously, the preferential orientation of the chains of the at least one polymer in one particular direction in the fiber is characterized by birefringence measurement [Kolbuk et al. "Optical birefringence and molecular orientation of electrospun polycaprolactone fibers by polarizing-interference microscopy" European Polymer Journal vol. 48 (2012): 275-83], X-ray fiber diffraction [Lafrance, C P et al. "Study of the distribution of molecular orientation in highly oriented polyethylene by x-ray diffraction" Macromolecules vol. 24 (1991): 4948-56], acoustic velocity [Moseley W W "The measurement of molecular orientation in fibers by acoustic methods" Journal of applied polymer science vol. 3 (1960): 266-76], polarized infrared (IR) spectroscopy [Boulet-Audet M et al. "Attenuated total reflection infrared spectroscopy: an efficient technique to quantitatively determine the orientation and conformation of proteins in single silk fibers" Applied spectroscopy vol. 62 (2008): 956-62], and polarized Raman spectroscopy [Rousseau M-E et al. "Study of protein conformation and orientation in silkworm and spider silk fibers using Raman microspectroscopy" Biomacromolecules vol. 5 (2004):2247-57].

More advantageously, the preferential orientation of the chains of the at least one polymer in one particular direction in the fiber is determined by wide-angle x-ray scattering measurements. It can be quantified by measuring the Hermans orientation factor [Lafrance, C P et al. "Study of the distribution of molecular orientation in highly oriented polyethylene by x-ray diffraction" Macromolecules vol. 24 (1991): 4948-56]. The Hermans orientation factor is a scalar descriptor, which quantifies the orientation of the normal to a diffraction plane with respect to a reference direction. If the fiber axis is chosen as the reference direction, this factor is null when the distribution is isotropic and ranges from -0.5 for a perfect orientation of the chains perpendicular to the fiber axis, to 1 for a perfect orientation of the chains parallel to the fiber axis.

In one embodiment, the at least two polymer chains of the polymer of each swollen fiber are oriented on a macromolecular scale in the swollen state with a preferential orientation in one direction. Preferably, the orientation of the at least two polymer chains of the polymer of each swollen fiber in one direction on a macromolecular scale is obtained by drying or by cooling below the glass transition temperature or melting temperature of some of the polymer segments during the spinning process of the polymer fiber and then swelling the fiber. Alternatively, the orientation of the at least two polymer chains of the swollen fiber in one direction on a macromolecular scale is obtained by stretching the dry fiber above the glass transition temperature and below the melting temperature of some of the polymer segments and then swelling the fiber. Alternatively, the orientation of the at least two polymer chains of the polymer of each swollen fiber in one direction on a macromolecular scale is obtained by stretching the swollen fiber and then drying it while maintaining the stretching and then swelling it again without the stretching.

Linear density

In one embodiment, the linear density of each of the at least two fibers in the dry state is greater than 0.001 mg/m (corresponding to 0.01 Dtex), preferably greater than 0.01 mg/m (corresponding to 0.1 Dtex), more preferably greater than 0.1 mg/m (corresponding to 1 Dtex), even more preferably greater than 1 mg/m (corresponding to 10 Dtex).
In another embodiment, the linear density of each of the at least two fibers in the swollen state measured at equilibrium swelling in the swelling agent at room temperature is greater than 0.01 mg/m (corresponding to 0.1 Dtex), preferably greater than 0.1 mg/m (corresponding to 1 Dtex), more preferably greater than 1 mg/m (corresponding to 10 Dtex), even more preferably greater than 10 mg/m (corresponding to 100 Dtex).

Diameter of the swollen fiber

Advantageously, the diameter of the fiber in the dry state ranges from 1 μm to 500 μm, preferably from 10 μm to 200 μm, more preferably from 15 μm to 150 μm, even more preferably it is of about 100 μm.
Advantageously, the diameter of the fiber in the dry state ranges from 1 μm to 500 μm, preferably from 10 μm to 200 μm, more preferably from 20 μm to 150 μm, even more preferably it is of about 80 μm.
Advantageously, the diameter of the fiber in the dry state ranges from 1 μm to 500 μm, preferably from 10 μm to 200 μm, more preferably from 20 μm to 150 μm, even more preferably it is of about 60 μm.
Advantageously, the diameter of the fiber in the dry state ranges from 1 μm to 500 μm, preferably from 10 μm to 200 μm, more preferably from 20 μm to 150 μm, even more preferably it is of about 40 μm.
Advantageously, the diameter of the fiber in the dry state ranges from 1 μm to 500 μm, preferably from 10 μm to 200 μm, more preferably from 20 μm to 150 μm, even more preferably it is of about 15 μm.
Advantageously, the diameter of the swollen fiber ranges from 1 μm to 1000 μm, preferably from 5 μm to 500 μm, more preferably from 10 μm to 200 μm, even more preferably from 15 μm to 150 μm.
Advantageously, each swollen fiber has a big and a small diameter, the small diameter of the swollen fiber ranges from 1 μm to 500 μm, preferably from 5 μm to 200 μm, more preferably from 10 μm to 100 μm, even more preferably from 15 μm to 80 μm ; and the big diameter of the swollen fiber ranges from 5 μm to 1000 μm, preferably from 20 μm to 500 μm, more preferably from 40 μm to 200 μm, even more preferably from 50 μm to 150 μm.

Swelling agent

Each swollen fiber comprises at least one swelling agent. The at least one swelling agent is suitable for swelling the polymer of the swollen fiber.
Advantageously, the at least one swelling agent is selected from the group consisting of: water, dimethyl sulfoxide (DMSO), dimethylformamide (DMF) and mixtures thereof.
If the polymer comprises or consists of poly(vinyl alcohol), then the at least one swelling agent is preferably selected from the group consisting of: water, dimethyl sulfoxide (DMSO), dimethylformamide (DMF) and mixtures thereof.
When the at least one swelling agent is water, the swollen fiber is a hydrogel fiber.
In one embodiment, the weight of the at least one swelling agent represents at least 10% of the total weight of each swollen fiber at room temperature, preferably the weight of the at least one swelling agent represents from 20% to 80% of the total weight of each swollen fiber at room temperature, more preferably the weight of the at least one swelling agent represents from 30% to 70% of the total weight of each swollen fiber at room temperature, even more preferably the weight of the at least one swelling agent represents about 50% of the total weight of each swollen fiber at room temperature.
In one embodiment, the at least one swelling agent is a solution or a suspension. In this case, the solvent in the swelling agent acts as swelling agent, whereas the solute or the suspended particles does not act as swelling agent. For example, the swelling agent may be an aqueous solution of PVA; in this case, the water acts as swelling agent, whereas the PVA solute is intended to cover the fiber, for example to improve the smoothness, improve the resistance to abrasion and increase the life of the fiber. For another example, the swelling agent may be a sodium chloride solution, in particular a sodium chloride solution comprising 9 g/L of NaCl; in this case, the water acts as swelling agent, whereas the NaCl solute is intended to act as an isotonic agent.

Process for obtaining the swollen fibers

Advantageously, the swollen fibers according to the invention are obtained by a process comprising a step of putting into contact a polymer fiber with at least one swelling agent.
More advantageously, the swollen fibers according to the invention may obtained by a process comprising the following steps:

a) injecting a solution comprising a polymer through a spinneret into a coagulation bath,
b) extruding the solvent from the solution comprising the polymer to obtain polymer fibers through precipitation,
c) washing, drying and drawing the polymer fibers obtained at step b), and
d) contacting the polymer fibers obtained at step c) with at least one swelling agent.

In one embodiment, the solution comprising a polymer in step a) is an aqueous solution of PVA.

In one embodiment, the solution comprising a polymer in step a) is a solution of PVA in DMSO.

In one embodiment, the solution comprising a polymer in step a) is a solution of PVA in a mixture of water and DMSO.

In one embodiment, the solution comprising a polymer in step a) is a solution of PVA having a PVA concentration ranging from 5 to 30 wt%, preferably ranging from 10 to 20 wt%.

In one embodiment, the solution comprising a polymer in step a) is injected through a spinneret which holes have diameters ranging from 10 to 500 μm, preferably ranging from 150 to 350 μm.

In one embodiment, the solution comprising a polymer in step a) is injected through a spinneret into a coagulation bath which is filled with a saline aqueous solution.

In one embodiment, the solution comprising a polymer in step a) is injected through a spinneret into a coagulation bath which is filled with ethanol, isopropanol or butanol.

In one embodiment, the drawing of polymer fibers at step c) is carried out at 150 °C. In one embodiment, the drawing of polymer fibers at step c) is carried out with a 21 m/min draw speed. In one embodiment, the drawing of polymer fibers at step c) is carried out at 150 °C with a 21 m/min draw speed.

In one embodiment, the drawing of polymer fibers at step c) is carried out at 50 °C. In one embodiment, the drawing of polymer fibers at step c) is carried out with a draw speed of about 1 m/min. In one embodiment, the drawing of polymer fibers at step c) is carried out at 50 °C with a draw speed of about 1 m/min.

The step of drawing polymer fibers at step c) allows to increase the orientation rate of the fibers and improve their strength and stiffness in tension.

In one embodiment, the step d) of immersing the polymer fibers obtained at step c) is carried out at room temperature. Preferably, the room temperature is 20°C.

In one embodiment, the step d) of immersing the polymer fibers obtained at step c) is carried out during at least 15 minutes, preferably during at least 30 minutes, more preferably during about 1 hour.

In one embodiment, the at least one swelling agent used in step d) is selected from the group consisting of water, dimethyl sulfoxide (DMSO), dimethylformamide (DMF) and mixtures thereof. Preferably, the at least one swelling agent used in step d) is water.

Form of the swollen fiber assembly

The at least two swollen fibers of the swollen fiber assembly according to the invention are either non-woven, twisted, woven, braided or knitted together, or included within a polymeric matrix further comprised in the fiber assembly.

In one embodiment, the at least two swollen fibers of the swollen fiber assembly are at least one of: twisted, non-woven, woven, braided, knitted together and any combinations thereof. In one preferred embodiment, the at least two swollen fibers of the swollen fiber assembly are twisted. In another preferred embodiment, the at least two swollen fibers of the swollen fiber assembly are braided and woven.

The twisting, non-weaving, weaving, braiding, knitting or inclusion within a polymeric matrix may concern part or all the length of the at least two swollen fibers of the swollen fiber assembly.

Advantageously, the swollen fiber assembly has a shape selected from the group consisting of: a twisted fabric such as a yarn or a rope, a non-woven fabric, a woven fabric, a braided fabric and a knitted fabric. Advantageously, the swollen fiber assembly has a shape of a twisted fabric such as a yarn or a rope. More advantageously, the swollen fiber assembly has a shape of a twisted yarn structure. Even more advantageously, the swollen fiber assembly has a shape of a twisted yarn structure having a twist ranging from 0.1 to 2.0 turn/cm. Even more advantageously, the swollen fiber assembly has a shape of a twisted yarn structure composed of a number of fibers ranging from 5 to 200. Alternatively, the swollen fiber assembly has a shape of a twisted double helix rope structure. More advantageously, the swollen fiber assembly has a shape of a twisted double helix rope structure in which the twist of the yarn ranges from 0.5 to 2.0 turns/cm. Even more advantageously, the swollen fiber assembly has a shape of a twisted double helix rope structure composed of a number of yarns ranging from 2 to 100, themselves composed of a number of fibers ranging from 5 to 300.

The inventors have surprisingly discovered that a swollen fiber and assemblies of thereof according to the invention are fatigue resistant, i.e. after being submitted to a mechanical stress, they recover all or part of their mechanical behavior (dimension, stiffness, hysteresis) by simply applying a rest time. When rest times are inserted, this recovery effect allows to reduce or even to suppress the creep and the loss of stiffness produced by the repetition of a large number of cycles (>$10^5$).

Advantageously, the swollen fiber assembly according to the invention comprises 1 to 24 strands, composed of 6 to 24 yarns, preferably 10 to 20 yarns, organized into bundles of 1 to 6 strands, preferably 2 to 4 strands, and twisted together with a twisting rate from 0.1 tr/cm to 10 tr/cm, preferably with a twisting rate from 0.2 tr/cm to 5 tr/cm, more preferably with a twisting rate from 0.2 tr/cm to 2 tr/cm, wherein each yarn comprises 5 to 300 fibers, preferably 10 to 100 fibers, more preferably 15 fibers, twisted together before assembly with a twisting rate from 0.1 to 2 tr/cm, preferably with a twisting rate of 0.4 to 1.5 tr/cm, more preferably with a twisting rate of 1.25 tr/cm.

Advantageously, the swollen fiber assembly according to the invention comprises 8 to 24 strands, preferably 12 to 16 strands, composed of 6 to 24 yarns, preferably 10 to 20 yarns, organized into bundles of 1 to 6 strands, preferably 2 to 4 strands, and parallel from each other, wherein each yarn comprises 10 to 1000 fibers, preferably 10 to 100 fibers, more preferably 15 fibers, twisted together before assembly with a twisting rate from 0.1 to 2 tr/cm, preferably with a twisting rate of 0.4 to 1.5 tr/cm, more preferably with a twisting rate of 1.25 tr/cm.

More advantageously, the swollen fiber assembly has a shape of a twisted yarn structure. Even more advantageously, the swollen fiber assembly has a shape of a twisted yarn structure having a twist ranging from 0.1 to 2.0 turn/cm. Even more advantageously, the swollen fiber assembly has a shape of a twisted yarn structure composed of a number of fibers ranging from 5 to 300. Alternatively, the swollen fiber assembly has a shape of a twisted double helix rope structure. More advantageously, the swollen fiber assembly has a shape of a twisted double helix rope structure in which the twist of the yarn ranges from 0.1 to 2.0 turns/cm. Even more advantageously, the swollen fiber assembly has a shape of a twisted double helix rope structure composed of a number of yarns ranging from 2 to 100, themselves composed of a number of fibers ranging from 5 to 300.

In another embodiment, the at least two fibers of the swollen fiber assembly according to the invention are included within a polymeric matrix further comprised in the swollen fiber assembly, wherein said polymeric matrix is not soluble in a swelling agent.

Advantageously, the swollen fiber assembly according to the invention has the shape of a tube, wherein the at least two fibers may have different orientations from each other with respect to the tube axis (which corresponds to the length direction of said tube).

Advantageously, the swollen fiber assembly according to the invention has the shape of a sheet, wherein the at least two fibers may have different orientations from each other within the plane of the sheet.

Advantageously, the swollen fiber assembly according to the invention has the shape of a block, wherein the block may have any geometry, such as a cube, a cuboid, a parallelepiped, a sphere, a disc, a cylinder, a cone or a pyramid.

In still another embodiment, the at least two fibers of the swollen fiber assembly according to the invention are non-woven, twisted, woven, braided or knitted together, wherein the resulting twisted fabric such as a non-woven fabric, a rope, a woven fabric, a braided fabric or a knitted fabric is included within a polymeric matrix further comprised in the swollen fiber assembly.

In one embodiment, the swollen fiber assembly is in the form of a yarn comprising from 5 to 300 swollen fibers, preferably from 10 to 100 swollen fibers. The swollen fibers may be either twisted or parallel in the yarn. Preferably, the swollen fibers are parallel to each other in the yarn or slightly twisted with a twist lower than 2 turns/cm.

## Polymeric matrix

In one embodiment, the swollen fiber assembly comprises at least two swollen fibers and a polymeric matrix entrapping the at least two fibers. The at least two swollen fibers may be either entrapped individually in the polymeric matrix, or entrapped in the form of a yarn of at least two swollen fibers in the polymeric matrix, or entrapped in the form of a twisted fabric such as a rope, a non-woven fabric, a woven fabric, a braided fabric or a knitted fabric in the polymeric matrix.

Advantageously, the polymeric matrix is made of a polymer selected from the group consisting of: poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and any copolymers thereof.

## Coating of the swollen fiber assembly

According to an embodiment, the swollen fiber assembly is coated with a polymer layer. Preferably, the polymer of said polymer layer is selected from the group consisting of poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and any copolymers thereof. More preferably, said polymer layer is a poly(vinyl alcohol) layer.

In one embodiment, the swollen fiber assembly is coated with a composite comprising a polymer and inorganic fillers. Advantageously, the inorganic fillers comprised in the coating are composed of hydroxyapatite, calcium phosphate or bioactive glass, which facilitates the local bone regeneration and therefore the integration of the assembly in bone

tissues.

In one embodiment, the coating of the swollen fiber assembly, in particular the poly(vinyl alcohol) coating, allows to reduce the friction against other surfaces and allows an easier insertion of the swollen fiber assembly in the body of the patient in need, and therefore it facilitates the gesture of the surgeon.

In one embodiment, the coating of the swollen fiber assembly, in particular the composite coating, allows to incorporate bioactive substances that favor the integration in the body and reduce the fibrous encapsulation induced by the inflammatory response.

Properties of the swollen fiber assembly

The inventors have surprisingly discovered that the swollen fibers and swollen fiber assemblies according to the present invention are able, after a cyclic mechanical stress, to recover their original mechanical behavior (dimension, stiffness, hysteresis) by simply applying a resting time.

Compared with the hydrogels of the prior art, the swollen fibers and swollen fiber assemblies according to the present invention have increased durability, allowing the maintenance of mechanical functionality (stability of the dimensions and stability of the mechanical behavior) over a longer period of use. In addition, the high anisotropy of the swollen fibers according to the present invention allows them to have a higher limit of elasticity than other so-called 'fatigue resistant' hydrogels. This elasticity over a large stress and strain range allows them to better resist to cyclic stresses or strains that are physiologically relevant.

Without wishing to be bound by any theory, it seems that the properties of semi-crystalline polymer microstructures having an orientation on a macromolecular scale in the swollen state allow the swollen fibers and swollen fiber assemblies according to the invention to have tensile properties close to those of biological tissues.

Moreover, it seems that the presence of weak reversible bonds in the amorphous parts of the polymer chains network of the swollen fiber allows for dissipative deformation and self-recovery upon rest (the dissociation and reassociation of such bonds upon mechanical solicitations seem to act as dissipative processes to reduce the irreversible damage of the fibers and increase their fatigue resistance). This leads to remarkable fatigue resistance under loading histories that are physiologically relevant.

The orientation on a macromolecular scale in the swollen state of the at least two polymer chains in association with the presence of weak bonds in the amorphous areas of the polymer of the swollen fiber allows to obtain swollen fibers and swollen fiber assemblies which have high tensile properties and enhanced fatigue resistance.

Such features of the swollen fibers according to the present invention may be used for manufacturing synthetic tissue substitutes and implants with high tensile properties and enhanced fatigue resistance.

**Use of the swollen fiber assembly**

Advantageously, the swollen fiber assembly according to the invention is used as or in implants (for example as or in prosthesis), as or in orthosis, as or in exoskeleton, or in a robotic structure.

In one embodiment, the swollen fiber assembly according to the invention is used for soft tissue reconstruction (orthopedics, cardiovascular, digestive...). For example, the fiber assemblies for soft tissue reconstruction may be:

- ligament substitutes,
- tissue reconstruction devices for soft tissues,
- tissue reinforcement devices (for example meshes for hernia repair),
- tissue stabilizers, i.e. a device that does not act as a ligament substitutes but is placed around a joint (fixed on the two bones which are articulated), to complete the reconstruction surgery by giving more stability.

In another embodiment, the swollen fiber assembly is used for soft robotics, especially in aqueous environments (underwater soft robots or surgical soft robots).

When sufficiently long rest periods (≥ 30 minutes, preferably ≥ 1 hour, more preferably ≥ 2 hours, even more preferably ≥ 8 hours) are inserted between periods of cyclic loading, the swollen fiber assembly according to the present invention allows:

- to reduce the residual elongation and the softening of the device under physiological conditions (repeated cyclic stress phases interspaced with rest phases) or cyclic loading of robots, and
- to maintain a stable mechanical behavior during a large number of loading cycles (> $10^5$ cycles).

Advantageously, the swollen fiber assembly is used *ex vivo* and/or *in vivo.* More advantageously, the swollen fiber assembly is used *in vivo.* Alternatively, the swollen fiber assembly is used *ex vivo* and/or *in vivo* but without

invasiveness in the mammal's body, in particular in the human's body.

**Process for recovering the original physical properties of the swollen fiber assembly**

The present invention also relates to a process for recovering the original properties of the swollen fiber, comprising the following steps:

- applying a constant mechanical stress or a loading-unloading cycle or a series of loading-unloading cycles on said swollen fiber, then
- applying a resting time of at least 30 minutes.

The present invention also relates to a process for recovering the original properties of the swollen fiber assembly, comprising the following steps:

- applying a constant mechanical stress or a loading-unloading cycle or a series of loading-unloading cycles on said swollen fiber assembly, then
- applying a resting time of at least 30 minutes.

In one embodiment, the resting time at step b) lasts at least 30 minutes, preferably at least 1 hour, more preferably at least 2 hours, even more preferably about 8 hours.

Indeed, the inventors have surprisingly discovered that when these swollen fibers or swollen fiber assemblies are subjected to cyclic loading in tension, they soften and elongate; but after a period of rest (without applied tension), these fibers recover their initial behavior (entirely or partially depending on the maximum stress/strain that was applied during the cycles). This is self-recovery phenomenon of the swollen fiber or swollen fiber assembly according to the invention.

In addition, such process allowing a self-recovery phenomenon of the swollen fiber or swollen fiber assembly according to the invention is compatible with the human daily cycle and persists after many cycles (at least $10^5$ cycles of mechanical stress- resting time). This allows the swollen fiber assemblies to mimic and/or replace mammal body tissues, as they have high tensile properties and enhanced fatigue resistance.

**BRIEF DESCRIPTION OF THE DRAWINGS**

**Figure 1** are pictures of yarns : (**A**) dry-spun yarn comprising 15 PVA fibers obtained by the "dry spinning" method, (**B**) wet-spun yarn comprising 100 PVA fibers obtained by the "wet spinning" method.

**Figure 2** shows images on the left of transmission optical microscopy observations of one dry-spun PVA fiber (**A**) and one wet-spun PVA fiber (**B**), both in the dry state. Images on the right are the same images obtained after thresholding.

**Figure 3** are images showing the cross-sections of dry-spun PVA fibers observed by Scanning Electron Microscopy (SEM): (**A**) in the initial dry state (state 1), (**B**) in the state obtained after swelling and drying (state 3).

**Figure 4** is the same as **Figure 3** for wet-spun PVA fibers.

**Figure 5** shows the thermograms obtained by Differential Scanning Calorimetry (DSC) for dry-spun PVA fibers (**A**: heating, **B**: cooling), wet-spun PVA fibers (**C**: heating, **D**: cooling), solvent-cast PVA films (**E**: heating, **F**: cooling).

**Figure 6** is a group of graphs showing the values of the crystallinity Xc of dry-spun PVA fibers, wet-spun PVA fibers and solvent-cast PVA films in the initial dry state (state 1): (**A**) Xc as measured by DSC; (**B**) Xc as measured by WAXS (White bars show the values for dry-spun and wet-spun fibers in state 3 for comparison).

**Figure 7** shows 2D diffraction images obtained by Wide-Angle X-Ray scattering (WAXS) for a dry-spun PVA yarn (**A**) and a wet-spun PVA yarn (**B**), both in the initial dry state (state 1).

**Figure 8** represents plots of the scattering intensity integrated over an entire quadrant as a function of the scattering angle 2$\theta$ for dry-spun yarn (**A**), wet-spun yarn(**B**) and solvent-cast film (**C**). All in the initial dry state (state 1).

**Figure 9** is a group of graphs showing the contraction rate (**A**) and the linear density (**B**) of the dry-spun and wet-spun

PVA yarns in different states: (1) the initial dry state, (2) the equilibrium swollen state in water at 20°C, (3) the dry state obtained after drying from state 2, (4) the equilibrium swollen state obtained after swelling again from state 3.

**Figure 10** is a graph showing the swelling ratio at equilibrium in water at 20°C (state 2) for dry-spun fibers, wet-spun fibers and solvent-cast films.

**Figure 11** shows 2D diffraction images obtained by synchrotron Wide-Angle X-Ray scattering (WAXS) for an individual dry-spun hydrogel yarn (**A**) and a wet-spun hydrogel yarn (**B**), both in the equilibrium swollen state at 20°C (state 2).

**Figure 12** represents plots of the scattered intensity integrated between $2\theta$ = 15.2° and 16.2° as a function of the azimuthal angle $\psi$ ($\psi$ = 0° in the direction of the fiber) for an individual dry-spun hydrogel yarn (**A**) and a wet-spun hydrogel yarn (**B**), both in the equilibrium swollen state (state 2). The corresponding values of the Hermans orientation factor $f_H$ are indicated on each graph.

**Figure 13** is a graph showing the tensile stress as a function of the tensile strain (stress-strain curves) for an individual dry-spun PVA fiber, a wet-spun PVA yarn and a solvent-cast PVA film, all in the equilibrium swollen state at 20°C (state 2). The end of the curves corresponds to fracture (protocol (i)). The gray area indicates the typical range of tensile responses for the human Anterior Cruciate Ligament (ACL).

**Figure 14** is a group of graphs showing the tensile stress-strain curves (gray curves) under cyclic loading with increasing strain amplitude (protocol (ii)) for an individual dry-spun fiber (**A**), a wet-spun yarn (**B**) and a solvent-cast film (**C**), all in the equilibrium swollen state at 20°C (state 2). Black curves show the tensile response until fracture (protocol (i)).

**Figure 15** is a graph showing the residual strain measured after each cycle of protocol (ii) as a function of the applied maximum stress.

**Figure 16** is a drawing representing the loading history applied to characterize the response to cyclic loading and the effect of rest time.

**Figure 17** is a group of graphs showing the effect of cyclic loading and rest on the tensile behavior of dry-spun PVA hydrogel fibers: (**A**) stress-strain curves for the cycles 1, 2 and 5; (**B**) stress-strain curves for the cycle 1 and the first cycle after a 2h rest time.

**Figure 18** is the same as **Figure 17** for wet-spun PVA hydrogel yarns.

**Figure 19** is the same as **Figure 17** for solvent-cast PVA hydrogel films.

**Figure 20** is a group of graphs showing the evolution of tensile properties during the five loading cycles before rest for dry-spun PVA hydrogel fibers, wet-spun PVA hydrogel yarns and solvent-cast PVA hydrogel films: (A) Young's modulus E normalized by the Young's modulus at the first loading E(cycle 1) as a function of the number of cycles; (**B**) Maximum applied stress $\sigma_{max}$ normalized by the maximum applied stress at the first loading $\sigma_{max}$(cycle 1) as a function of the number of cycles.

**Figure 21** is a group of graphs showing the evolution of tensile properties as a function of rest time for dry-spun PVA hydrogel fibers, wet-spun PVA hydrogel yarns and solvent-cast PVA hydrogel films: (**A**) Young's modulus E normalized by the Young's modulus at the first loading E(cycle 1); (**B**) Maximum applied stress $\sigma_{max}$ normalized by the maximum applied stress at the first loading $\sigma_{max}$(cycle 1).

**Figure 22** is a group of graphs showing the effect of cyclic loading and rest on the tensile behavior of wet-spun PVA hydrogel yarns for a modified testing protocol applying a constant maximum stress (1.6 MPa) in each cycle: (**A**) stress-strain curves for the cycles 1, 2 and 5; (**B**) stress-strain curves for the cycle 1 and the first cycle after a 2h rest time.

**Figure 23** shows the evolution of the diameter of one dry-spun PVA hydrogel fiber during cyclic loading between 0% and 60% in water at room temperature: (**A**) pictures of the fiber during the first loading cycle at strain = 0%, 30% and 60%; (**B**) graph showing the diameter of the fiber as a function of the tensile strain during the 1$^{st}$ and 5$^{th}$ loading cycles.

**Figure 24** shows 2D diffraction images obtained by synchrotron Wide-Angle X-Ray scattering (WAXS) for an individual dry-spun hydrogel yarn in the following states: (**A**) initial equilibrium swelling state at 0% strain, (**B**) under a maximum strain of 40% during the 1st loading cycle, (**C**) under 10% strain after 2 loading cycles, (**D**) after 1 h rest under 10% strain.

**Figure 25** represents intensity plots from the 2D WAXS patterns of Figure 24: (**A**) intensity integrated over an entire quadrant as a function of scattering angle $2\theta$; (**B**) intensity integrated between and $2\theta = 15.2°$ and 16.2° as a function of the azimuthal angle $\psi$ ($\psi = 0°$ in the direction of the fiber). The values of intensity are multiplied by $(1+\varepsilon)$ to correct for the variation in scattering volume as a function of tensile strain $\varepsilon$.

**Figure 26** is a drawing representing the loading history applied to characterize the response to a large number of loading-unloading cycles (fatigue cycles) and the effect of rest time. Testing was performed in water at 37°C.

**Figure 27** is a group of graphs showing the evolution of the tensile properties measured just after rest times (cycles A) as a function of the number of fatigue cycles for dry-spun PVA hydrogel fibers: (**A**) normalized residual strain; (**B**) normalized Young's modulus; (**C**) normalized force at 35% strain; (**D**) normalized hysteresis.

**Figure 28** is a group of graphs showing the evolution of the tensile properties measured just after a series of $10^4$ fatigue cycles (cycles B) as a function of the number of fatigue cycles for dry-spun PVA hydrogel fibers: (**A**) normalized residual strain; (**B**) normalized Young's modulus; (**C**) normalized force at 35% strain; (**D**) normalized hysteresis.

**Figure 29** is a graph showing the stress-strain curves measured just after rest times (cycles A) for one dry-spun PVA hydrogel fibers. The superimposed curves correspond to the first cycle A and all the subsequent cycles A after $10^4$, $2 \cdot 10^4$, $3 \cdot 10^4$, $4 \cdot 10^4$, $5 \cdot 10^4$, $6 \cdot 10^4$, $7 \cdot 10^4$ $8 \cdot 10^4$, $9 \cdot 10^4$, $10^5$, $1.1 \cdot 10^5$ fatigue cycles.

## EXAMPLES

The present invention is further illustrated by the following examples.

<u>Example 1: Preparation processes of poly(vinyl alcohol) (PVA) dry fibers in yarns comprising 15 and 100 PVA fibers, and of PVA solvent cast films for comparison.</u>

### Materials and Methods

Dry-spun PVA fibers were produced in the form of yarns by a dry spinning method:

- an aqueous solution of PVA having a hydrolysis degree greater than 95% was injected through a spinneret;
- once the solution has passed through the spinneret (15 holes), the PVA fibers being formed were subjected to a flow of air or other hot gas to evaporate the swelling agent and solidify the fibers;
- the resulting fibers were then caught by a recovery roller and fed to the rest of the spinning line, where the 15 fibers were slightly twisted together to form one yarn.

Wet-spun PVA fibers were produced in the form of yarns by a wet spinning method:

- a PVA solution (Mowiol® 56-98, molecular weight Mw = 195 000 g/mol, hydrolysis degree greater than 98%, 13% w/w of PVA relative to the weight of the solution) is injected through a spinneret (100 holes having a 70 μm diameter) with a 4 m/min speed of injection and into a coagulation bath which is filed with a saline aqueous solution (300 g/L $Na_2SO_4$ + 10 g/L NaOH);
- the water is extruded from the PVA solution to obtain PVA fibers through precipitation;
- the PVA fibers are then washed and dried,
- the PVA fibers are then drawn at 150 °C with a 21 m/min draw speed and then caught by a recovery roller and fed to the rest of the spinning line, where 100 fibers were assembled to form one yarn.

Isotropic films of PVA were produced by a solvent cast process for the sake of comparison. For that, an aqueous solution of PVA (Sigma-Aldrich, molecular weight Mw = 89 000- 98 000 g/mol, hydrolysis degree greater than 99%, 10% w/w of PVA relative to the weight of the solution) was prepared by dissolving PVA powder in distilled water at 95°C for 1 hour. The PVA solution was poured in Petri dishes up to a 5 mm thickness and left to evaporate at room temperature for 72 h. After that time, dry PVA films were collected.

**Results**

The yarn of dry-spun PVA fibers comprising 15 dry PVA fibers was slightly twisted with a twist of about 1.25 turn/cm. Its diameter as wound on the reel was of about 350 ± 25 μm (see picture in **Figure 1A**).

The yarn of wet-spun PVA fibers comprising 100 dry PVA fibers had almost parallel fibers with a twist lower than 0.5 turn/cm. When wound on the reel, the yarn was flattened with a width of about 1 mm and a thickness of about 0.1 mm (see picture in **Figure 1B**).

The dry solvent-cast PVA films had a thickness of about 0.5 mm.

Example 2: Physical properties of the dry PVA fibers and films obtained in Example 1

**Materials and Methods**

The PVA yarns and films of Example 1 were used.

The mean diameters of dry PVA dry-spun and wet-spun fibers were assessed by transmission optical microscopy on an Axio Scope A1 instrument (Zeiss). Their values were determined by image analysis with the software ImageJ. Images were binarized and the mean diameter was obtained by dividing the surface area of the fiber by its length. Measurements were performed on at least five different sites on at least five different dry fibers. The PVA dry fibers were also analyzed via Scanning Electron Microscopy (SEM) on a Nova Nano 450 instrument (FEI).

The linear density of the dry PVA fibers were measured by weighting a given length of yarn in the dry state using a precision balance. The value of the linear density of the PVA dry fibers were obtained by dividing this value by the number of fibers per yarn (dry-spun: 15 fibers/yarn, wet-spun: 100 fibers/yarn).

The crystallinity, glass-transition and melting temperatures were measured by differential scanning calorimetry (DSC) on a Q2000 instrument (TA instrument). Dry fiber samples weighing between 2 and 3 mg were cut and sealed in aluminum crucibles. Thermograms were obtained during heating at 10°C/min from 0°C to 250°C and then cooling at the same rate from 250°C to 0°C. The melting temperature and the crystallinity were extracted from the thermograms. The melting temperature ($T_m$) corresponds to the maximum of the melting peak. The crystallinity (Xc) is given by $Xc = \Delta H / \Delta Hc$ where ΔH is the enthalpy of fusion which corresponds to the area under the melting peak of the sample and ΔHc is the enthalpy of fusion of the pure PVA crystal (ΔHc = 138.6 J/g) [Peppas, N. A. et al. "Crystallization kinetics of poly(vinyl alcohol)" Journal of applied polymer science vol. 27 (1982): 4787-4797]. The presence of residual water in the fibers is estimated by considering the enthalpy of vaporization of water ΔHv = 2258 J/g.

The crystallinity of the fibers and the films in the dry state were measured by analyzing the fibers by wide angle X-ray scattering (WAXS). These measurements were performed on a Nano-inXider. The fibers were aligned parallel to each other and positioned on a cardboard support inserted into the sample holder of the Nano-inXider. Acquisitions with different sample orientations were performed and then reconstructed into a 2D image with an azimuthal domain of 180°. The angular step used was $\Delta\Psi$= 18°, thus 11 positions were analyzed with a time of 900 seconds per position.

**Results**

The mean diameter of the dry-spun and wet-spun PVA fibers in the dry state was assessed from optical micrographs as shown in **Figure 2A** for dry-spun and **Figure 2B** for wet-spun fibers. The mean diameters were 80±8 μm and 28±5 μm for dry-spun and wet-spun fibers, respectively. Image analysis of different samples (>10) showed variations of the PVA dry fiber mean diameter of about 10% for the dry-spun fibers and more than 15% for the wet-spun fibers. These variations can be explained by the fact that the PVA dry fibers do not have a circular cross-section, as shown in the SEM observations of **Figure 3A** and **Figure 4A** for dry-spun and wet-spun fibers, respectively.

The linear density of the yarns in the dry state were 81±1 mg/m and 51±1 mg/m, for the dry-spun and wet-spun yarns, respectively. The corresponding linear densities of the fibers in the dry state were about 5 mg/m (50 Dtex) for the dry-spun fibers and about 0.5 mg/m (5 Dtex) for the wet-spun fibers.

The thermograms of the semi-crystalline structures of the fibers and films described in Example 1 are shown in **Figure 5**. For the dry-spun fibers, the melting temperature ($T_m$) during the first heating was 233°C for an enthalpy of fusion of ΔH=82 J/g, i.e. a crystallinity of 60±10 % (**Figure 5A**). In addition, a water vaporization peak was observed around 100°C with a total water vaporization enthalpy of ΔH=84 J/g. This corresponds to a presence of 3%(g/g) water in the sample coming from the water vapor present in the air. The glass transition temperature $T_g$ measured from the cooling thermogram was 75°C (**Figure 5B**). For the wet-spun fibers, the melting temperature ($T_m$) during the first heating was 226°C for an enthalpy of fusion of ΔH=73 J/g, i.e. a crystallinity of 53±10 % (**Figure 5C**). A water vaporization peak was also observed around 100°C with a total water vaporization enthalpy of ΔH=80 J/g, which corresponds to a presence of 3% (g/g) water. The glass transition temperature $T_g$ measured from the cooling thermogram was 75°C (**Figure 5D**). For the solvent-cast films, the melting temperature ($T_m$) during the first heating was 227°C for an enthalpy of fusion of ΔH=65 J/g,

i.e. a crystallinity of 47±10 % (**Figure 5E**). A larger water vaporization peak was observed with a total water vaporization enthalpy of ΔH=218 J/g, which corresponds to a presence of 10%(g/g) water. The glass transition temperature $T_g$ measured from the cooling thermogram was 72°C (**Figure 5F**). Values of crystallinity (Xc) measured by DSC are reported in **Figure 6A**.

The semi-crystalline microstructures of the dry-spun and wet-spun fibers in the dry state were characterized by WAXS. On the 2D diffraction patterns shown in **Figure 7A** and B, the peaks of the characteristic crystal planes of PVA were identified for both dry-spun and wet-spun fibers, and in particular the overlapping peaks of 101 and 101 planes which contain the axis of the polymer chains, i.e. the direction of the polymer backbone. The stronger intensity of these peaks in the direction perpendicular to the fiber axis indicates that the polymer chains are predominantly oriented along the fiber axis. In the case of the wet-spun fibers, additional diffraction peaks were observed and correspond to the presence of residual salts coming from the coagulation bath. In addition, the 2θ intensity profiles obtained by integrating the diffraction patterns over an entire quadrant (**Figure 8**) showed the expected position of the peaks of the $10\bar{1}$ and 101 planes at 19.5°, the 100 plane at 11°, and the 200 plane at 22.8°. Deconvolution fits were performed to measure the scattering coming from the amorphous and crystalline phases. The fitted peaks produced by the crystalline and amorphous phases are represented in **Figure 8** by full and dashed gray lines, respectively. The ratio between the area of the crystalline peaks over the total scattered area gives the value of the crystallinity as follows: Xc = 31% for dry-spun fibers, Xc = 25% for wet-spun fibers and Xc = 20% for the solvent-cast films **(Figure 6B).**

<u>Example 3: Preparation and physical characterization of PVA hydrogel fibers and films obtained by swelling in water of the dry PVA fibers and films of Example 1.</u>

**Materials and Methods**

The PVA dry yarns (=state 1) manufactured in Example 1 (each comprising 15 and 100 PVA dry fibers for dry-spun and wet-spun yarns, respectively) were immersed in demineralized water at room temperature and were left to swell during 1 hour before any measurement was performed (first swelling phase). At this stage (=state 2), the swelling equilibrium was considered to be reached (less than 5% variation in weight between 30 min and 60 min immersion time) and PVA hydrogel yarns comprising PVA hydrogel fibers were obtained. Then, these hydrogel fibers were left to dry for 72 h in open air at room temperature (drying phase) (=state 3). Finally, the yarns were immersed again in demineralized water at room temperature and they were left to swell during 1 hour again (second swelling phase) (=state 4).

The PVA dry films (state 1) manufactured in Example 1 were immersed in demineralized water at room temperature. Because they are thicker than fibers, they were left to swell during 24 hours before any measurement was performed (first swelling phase). At this stage (=state 2), the swelling equilibrium was considered to be reached (less than 5% variation in weight between 4 h and 6 h immersion time) and PVA hydrogel films were obtained.

For dry-spun and wet-spun fibers, the length of the yarns was measured in each state. The contraction ratio of the yarns (Y) in a given state is given by $Y = L/L_1$ where L is the length of the yarn in a given state and $L_1$ is the initial length of the yarn (=state 1).

For the dry-spun fibers, the mean diameter in the hydrogel state (state 2) was assessed by transmission optical microscopy on an Axio Scope A1 instrument (Zeiss) like for the dry fibers (see Materials and Methods in Example 1).

For both dry-spun and wet-spun fibers, the shape of the fiber cross-sections was characterized in the initially dry state (state 1) and the re-dried state (state 3) by Scanning Electron Microscopy (SEM) on a Nova Nano 450 instrument (FEI) at 5 kV using a secondary electron detector. The values of the small and large diameters were determined by image analysis with the software ImageJ.

The linear density of PVA fibers in a given state was obtained by weighting a given length of yarn in a given state using a precision balance and dividing this weight by this length and by the number of fibers composing the yarn.

The potential presence of an extractable soluble fraction in the PVA fibers was assessed by drying PVA fibers which have been swollen in a large volume of water (32 mg of fibers in 200 mL of water). The initial mass $m_1$ of the PVA dry fibers (=state 1) and their mass $m_3$ after swelling and drying (=state 3) were measured. The value of the extractable soluble fraction is given by $1 - (m_3/m_1)$.

The swelling ratio at equilibrium of the PVA hydrogel yarns and films (Q) is given by $Q = m_2/m_1$ where $m_2$ is the mass of the PVA hydrogel yarn or film after the first swelling (when the swelling equilibrium is reached) (=state 2) and $m_1$ is the initial mass of the PVA dry yarn or film (=state 1).

The crystallinity of the fibers after swelling and redrying (state 3) was measured by wide angle X-ray scattering (WAXS) using the same protocol as in Example 2.

**Results**

Upon immersion in the swelling agent (water), the length and diameter of the fibers changed. **Figure 9A** shows

the variation in length (contraction) of the yarns in the different states. For the first immersion, a contraction occurred along the fiber axis within the first minutes both for the dry-spun and the wet-spun fibers. After about 20 min, the length remained stable. At equilibrium swelling (state 2), the contraction rate (Y) was 71±1% and 37±1% for the dry-spun and wet-spun fibers, respectively. After drying (state 3), the fibers contracted more to reach Y = 68±1% and 30±1% for the dry-spun and wet-spun fibers, respectively. After the second immersion (state 4), the yarns elongated and reached the same lengths as in the ones obtained after the first immersion (state 2). The irreversible contraction observed during the first swelling can be attributed to a relaxation of some of the macromolecular orientation that was produced during spinning by stretching and cooling the fibers below the glass transition temperature of PVA.

The linear densities of the dry-spun and wet-spun fibers are shown in **Figure 9B** for the different states. After the first swelling phase (when the swelling equilibrium was reached) (=state 2), the linear densities of the hydrogel fibers were 16±1 mg/m (160 Dtex) and 6.6±1 mg/m (66 Dtex) for the dry-spun and wet-spun fibers, respectively.

After the drying phase (=state 3), the linear density of the fibers were 8±1 mg/m (80 Dtex) and 1.7±1 mg/m (17 Dtex) for the dry-spun and wet-spun fibers, respectively. These values are greater than the values of the linear density in the initial dry stage (=state 1: 5 mg/m for dry-spun fibers and 0.5 mg/m for wet-spun fibers). This difference between states 1 and 3 is explained by the irreversible contraction of the fibers occurring during the first swelling.

After the second swelling phase (=state 4), the PVA hydrogel fibers recovered the same linear densities as the ones obtained after the first swelling phase (state 2), i.e., about 16±1 mg/m (160 Dtex) and 6.9±1 mg/m (69 Dtex) for the dry-spun and wet-spun fibers, respectively.

The initial mass of the PVA dry fibers (=state 1) and their mass after drying (=state 3) the corresponding hydrogel fibers were similar. The extractable soluble fraction in both dry-spun and wet-spun PVA fibers was less than 5% and considered to be negligible.

The swelling ratios (Q) at equilibrium in water at room temperature (state 2) are shown in **Figure 10.** For the dry-spun fibers, Q = 2.1±0.3, which corresponds to a polymer mass concentration of 48±7% in the hydrogel state. For the wet-spun fibers, Q = 4.6±0.3, which corresponds to a polymer mass concentration of 21±2% in the hydrogel state. For the solvent cast films, Q = 2.6±0.3, which corresponds to a polymer mass concentration of 38±5% in the hydrogel state.

The change in diameter produced by swelling was measured by optical microscopy on individual fibers for dry-spun fibers. The mean diameter increased from 80±8 μm (state 1) to 115±5 μm (state 2).

The change in diameter between the initially dry state (state 1) and the swollen-redried state (state 3) was measured on individual fibers from SEM images (see **Figure 3B** and **4B** for dry-spun and wet-spun fibers, respectively). For the dry-spun fibers, the small diameter increased from 63±2 μm (state 1) to 72±2 μm (state 3) and the large diameter increased from 80±8 μm (state 1) to 102±2 μm (state 3). For the wet-spun fibers, the small diameter increased from 11±2 μm (state 1) to 23±2 μm (state 3) and the large diameter increased from 33±2 μm (state 1) to 66±5 μm (state 3).

The value of the small and large diameters in the swollen hydrogel state (states 2 and 4) can be assessed by assuming that swelling from the redried state (state 3) is isotropic. Dimensions in the swollen hydrogel state can thus be approximated by multiplying the dimensions in state 3 by $Q^{1/3}$, as follows. For the dry-spun fibers, the small and large diameters in hydrogel state are 50±4 μm and 71±5 μm, respectively. For the wet-spun fibers, the small and large diameters in hydrogel state are 35±4 μm and 101±9 μm, respectively.

The crystallinities of the dry-spun and wet-spun fibers after swelling and drying (state 3) were measured by WAXS and were very similar to the values in state 1, as shown in **Figure 6B**. This result is consistent with the idea that PVA crystallites do not melt upon swelling and that the fraction of PVA chains involved in crystallites is comparable in the dry and hydrogel states.

<u>Example 4: Orientation at the macromolecular scale in the swollen state of the PVA hydrogel fibers obtained by swelling the dry PVA fibers of Example 1.</u>

**Materials and Methods**

The dry-spun and wet-spun PVA yarns of Example 1 were used. In the case of dry-spun fibers, each experiment was performed on one individual fiber which was separated from the yarn. In the case of wet-spun fibers, each experiment was performed on one yarn comprising 100 fibers.

The orientation at the macromolecular scale in the swollen state was characterized by WAXS. Measurements were performed on a synchrotron beam line (DiffAbs line, SOLEIL, France) at an energy of 10 keV. In each experiment, one dry fiber or one dry yarn was placed in a customized sample holder between two mica windows and were hold vertically to remain on the path of the X-ray beam. The sample holder was filled with water and the sample was left to swell during 15 min free of any tension. The sample was then aligned with the X-ray beam and a 2D WAXS patterns was recorded in this swollen state.

The Hermans factor $f_H$ characterizing the macromolecular orientation was calculated from 2D WAXS patterns through the formula:

$$f_H = 1 - 3\left(\int_0^{\pi} I(\psi)\cdot(\cos\psi)^2\cdot\sin\psi\cdot d\psi\right)\Big/\left(\int_0^{\pi} I(\psi)\cdot\sin\psi\cdot d\psi\right)$$

where $\psi$ is the azimuthal angle with $\psi$ = 0° in the direction of the fiber, $I(\psi)$ is the mean intensity at angle $\psi$ averaged in the vicinity of the amorphous, 101 and $10\overline{1}$ peaks between scattering angles $2\theta$ = 15.2° and 16.2°.

**Results**

**Figures 11A** and B show the 2D WAXS patterns in the swollen state for the dry-spun and wet-spun hydrogel fibers, respectively. On these patterns, the concentration of the scattering 101 and $10\overline{1}$ peaks in the direction perpendicular to the fibers axis demonstrates that the fibers are oriented at the macromolecular scale in the swollen state.

The scattered intensity integrated in the vicinity of the amorphous, 101 and $10\overline{1}$ peaks was plotted as a function of the azimuthal angle $\psi$ ($\psi$ = 0° in the direction of the fiber) (**Figure 12**). The values of the corresponding Hermans orientation factor are $f_H$ = 0.57 for the dry-spun hydrogel fiber and 0.25 for the wet-spun hydrogel yarn. Such values greater than 0 indicate that the axis of the polymer chains is preferentially oriented parallel to the fibers axis and that this orientation is greater in the dry-spun fibers than the wet-spun yarn.

Example 5: Mechanical properties of the PVA hydrogel fibers and films obtained by swelling the dry fibers and films of Example 1.

**Materials and Methods**

The PVA yarns and films of Example 1 were swollen in demineralized water at room temperature and were left to swell free of any tension during at least 1 hour for the yarns and during 24 h for the films. In the case of dry-spun fibers, individual fibers were separated from the yarn for mechanical testing.

The tensile behavior of the hydrogel fibers and films were characterized using a 10 kN Allround testing machine (ZwickRoell) equipped with a testing chamber filled with water at controlled temperature. Measurements were performed in water at room temperature on individual fibers for the dry-spun PVA fibers, on yarns comprising 100 fibers for the wet-spun PVA fibers and on rectangular ribbons for the PVA solvent cast films. Tensile force was measured using a 10 N load cell for the dry-spun fibers and a 500 N load cell for the wet-spun yarns and the solvent cast films.

For all the experiments, the samples were stretched and unstretched along their long axis at a speed of 1 mm/s, which corresponds to a nominal strain rate of $9\pm3\ s^{-1}$.

The nominal tensile strain and stress were used. The nominal tensile strain ($\varepsilon$) is given by $\varepsilon = (L-L_0)/L_0$ where $L_0$ is the initial length of the sample determined by applying a small force (5±3 mN) and L is the length in a given tensile state. For the fibers and yarns, the nominal tensile stress ($\sigma$) is given by $\sigma = \rho\cdot F/m$ where F is the tensile force (N), m is the linear density of the fiber or yarn (g/m), and $\rho$ is the density of the samples ($\rho = 10^6\ g/m^3$ for the studied hydrogels (=state 2)). For the films, it is given by $\sigma = F/w\cdot t$ where F is the tensile force, w and t are the sample width and thickness, respectively.

Two protocols of tensile tests were performed with:

- (i) a simple loading until fracture;
- (ii) a series of loading-unloading between 0% strain and an increasing maximum strain, where the strain increment was 5% until 20% tensile strain, then 10% until 40% tensile strain and finally 20% until fracture.

In protocol (ii), the residual strain was measured as the highest strain for which the force decreased below 0.005 N during the unloading phase of each cycle.

**Results**

**Figure 13** shows the stress-strain curves until fracture (protocol (i)). The stress at break was 33±5 MPa for the dry-spun hydrogel fibers, 8±2 MPa for the wet-spun hydrogel yarns and 1.6±0.5 MPa for the hydrogel films.

The strain at break was 100±20% for the dry-spun hydrogel fibers, 250±50% for the wet-spun hydrogel yarns and 300±50% for the hydrogel films.

It is interesting to notice that both the dry-spun hydrogel fibers and wet-spun hydrogel yarns exhibit a much stiffer and more non-linear response than the solvent-cast hydrogel films, while having comparable (for dry-spun fibers) or even higher (for wet-spun yarns) swelling ratio (see **Figure 10**). Considering that the crystallinity of the fibers and films are close (see **Figure 6**), this stiffer and more non-linear response is attributed to the anisotropy of the macromolecular network composing the hydrogels. In the case of the dry-spun hydrogel fibers, the tensile response is close to that of human

ligament tissues, as indicated in **Figure 13** by the typical range of tensile responses for the human Anterior Cruciate Ligament (ACL) (Woo et al. "Tensile properties of the human femur-anterior cruciate ligament-tibia complex The effects of specimen age and orientation" Am. J. Sports Med., 1991, 19(3), pp. 217-225).

**Figure 14** compares the tensile response until fracture (protocol (i)) to the tensile response under cyclic loading with increasing strain amplitude (protocol (ii)). For all the samples, a Mullins-like effect was observed. Different regimes are found: under small maximum strain (about less than 10%), the behavior is almost perfectly elastic; under intermediate maximum strains, the behavior is non-linear elastic with a significant hysteresis; and under large maximum strains, the behavior is still non-linear elastic and a residual deformation appears.

**Figure 15** shows the residual strain measured after each cycle during protocol (ii) as a function of the maximum stress applied during the cycle. It clearly appears that the hydrogel fibers and yarns remain elastic over larger stress range than hydrogel films. For instance, the tensile stress required to produce a 20% residual strain was about 0.9 MPa for the hydrogel films while it was about 2 MPa for the wet-spun hydrogel fibers and about 30 MPa for the dry-spun hydrogel fibers. These results indicate that a higher macromolecular orientation provides a mean to increase very substantially the limit of elasticity. This effect supports the hypothesis that the swollen fibers and assemblies according to the invention should exhibit higher fatigue resistance.

<u>Example 6: Effect of cyclic tensile loading and effect of rest on the behavior of PVA hydrogel fibers and films obtained by swelling the dry fibers and films of Example 1.</u>

## Materials and Methods

The PVA yarns and films of Example 1 were swollen in demineralized water at room temperature and were left to swell free of any tension during at least 1 hour for the yarns and during 24 h for the films. In the case of dry-spun fibers, individual fibers were separated from the yarn for mechanical testing.

The same preparation and testing test-up as in Example 5 were used.

A series of cyclic tensile tests with rest time were performed to assess the presence of a self-recovery effect, as depicted in **Figure 16**. First, the samples were subjected to five loading cycles between 10% and a given maximum tensile strain. Then, they were left to rest in water free of any tension for a given period of time. A new loading cycle was performed again to assess the effect of rest. The following rest times were applied : 5 min, 15 min, 30 min, 1 h, 2 h.

The value of the maximum tensile strain applied during each cycle was selected to be close to the limit of elasticity of each sample. These values were determined from the results of protocol (ii) in Example 5 and correspond to the onset of residual strain (>5%) as indicated by the dotted line in **Figure 15**. It was 40% for the dry-spun hydrogel fibers, 120% for the wet-spun hydrogel yarns and 80% for the hydrogel films.

The protocol was modified to apply a same maximum tensile stress for each loading cycle. Experiments were performed with this modified protocol for wet-spun hydrogel yarns. The maximum applied stress was 1.7 MPa (close to the limit of elasticity determined in **Figure 15**).

The Young's modulus E was calculated for each loading stress-strain curve by measuring the slope of a linear fit between a strain of 5% and 20%.

## Results

The stress-strain response for the first five cycles of protocol (iii) (before a rest time was applied) is shown in **Figures 17A**, **18A** and **19A** for the dry-spun hydrogel fiber, the wet-spun hydrogel yarn and the hydrogel film, respectively. For all the samples, a significant hysteresis was observed during the first loading cycle but not during the subsequent loading cycles.

A softening of the tensile response was observed which is characterized by a decrease in the Young's modulus with the number of cycles (**Figure 20A**). For the dry-spun hydrogel fiber and the hydrogel film, the decay in Young's modulus was mainly done after the first cycle and progressed more slowly after the subsequent cycles. On the contrary, the Young's modulus of the wet-spun hydrogel yarn was little affected after the first cycle and decreased more significantly during the subsequent cycles. After 5 cycles, the Young's modulus was decreased by about 25% for the dry-spun hydrogel fiber and the wet-spun hydrogel yarn, while it was decreased by about 40% for the hydrogel film.

The softening of the tensile response is also characterized by a decrease in the maximum stress (**Figure 20B**) with the number of cycles. For all the samples, this decrease is more pronounced after the first cycle. Both the dry-spun hydrogel fiber and the wet-spun hydrogel yarn exhibited a similar decay reaching a 10% reduction after 5 cycles. This decay was slightly stronger in the case of the hydrogel film (15% reduction after 5 cycles).

The effect of a 2 h rest immerged in water is shown in **Figures 17B**, **18B** and **19B** for the dry-spun hydrogel fiber, the wet-spun hydrogel yarn and the hydrogel film, respectively. Within the experimental error, a full recovery of the initial mechanical response was clearly observed for the dry-spun hydrogel fiber (**Figure 17B**) and the wet-spun hydrogel yarn

(**Figure 18B**) while it was almost inexistent for the hydrogel film (**Figure 19B**).

The mechanical work applied per cycle can be assessed by measuring the area of the loading stress-strain curves. It is interesting to notice that the dry-spun and wet-spun hydrogel fibers exhibited a full-recovery ability for applied mechanical works that are comparable to (about 0.3 mJ/ $mm^3$ for the wet-spun yarn) or even one order of magnitude larger (about 2 $mJ/mm^3$ for the dry-spun fiber) than the work applied to the hydrogel film (about 0.2 $mJ/mm^3$).

The kinetics of the self-recovery was studied by varying the rest time, from 5 minutes to 2 hours. The evolutions of the normalized Young's modulus and normalized maximum applied stress as a function of rest time are shown in **Figure 21A** and B, respectively. For both the dry-spun hydrogel fiber and the wet-spun hydrogel yarn, a progressive recovery was achieved and was complete after 1-2h. In the case of the wet-spun hydrogel yarn, the recovery was complete within 5min for the Young's modulus but more gradual for the maximum applied stress. An opposite trend was obtained for the hydrogel film: no recovery of the Young's modulus and maximum applied stress was observed. A softening of the response was even measured, most likely due to the repeated loading cycles between each rest time.

In the protocol above, a given maximum strain was applied repeatedly. The ability of the hydrogel fibers and yarns to recover their initial mechanical properties upon rest is also efficient when a given maximum stress is applied repeatedly. This is illustrated in **Figure 22** in the case of a wet-spun hydrogel yarn. A softening of the response and an increase in the maximum strain was observed under the effect of five loading-unloading cycles between 0 and 1.6 MPa (**Figure 22A**). After a 2h rest, the initial tensile response was fully recovered (**Figure 22B**).

<u>Example 7: Role of hydrogen bonds in the dissipation and the recovery of hydrogel fibers obtained by swelling the dry fibers of Example 1.</u>

**Materials and Methods**

The dry-spun PVA yarns of Example 1 were used. Each experiment was performed on individual fibers which were separated from the yarn.

Five loading cycles in water at room temperature were applied to one hydrogel fiber between 0% and 60% strain using the same set-up and protocol as in Example 6. Pictures of the fiber were recorded using a long-range optical microscope. The evolution of the fiber diameter during cyclic loading was measured by image analysis using ImageJ.

The evolution of crystallinity and crystalline orientation during cyclic loading was characterized by in situ tensile measurements on a synchrotron beam line (DiffAbs line, SOLEIL, France) at an energy of 10 keV, like in Example 4. In each experiment, one dry fiber was placed in a customized sample holder between two mica windows and were hold vertically to remain on the path of the X-ray beam with one end of the fiber fixed onto the sample holder and the other end of the fiber attached to a stiff wire connected to a motorized translation stage. The sample holder was filled with water and the fiber was left to swell during 15 min free of any tension.

Experiments were performed in water at room temperature with the following steps:

- (i) The swollen fiber was aligned with the X-ray beam and its length was taken as the initial length at rest (strain = 0%). One 2D WAXS pattern was recorded.
- (ii) The swollen fiber was stretched and unstretched twice between 0% and 40% strain. 2D WAXS pattern were recorded at 0% and 40% during each cycle.
- (iii) The swollen fiber was left to rest in water free of any tension for 1 h.
- (iv) After the rest time, the swollen fiber was aligned again into the initial position (0% strain) to record one 2D WAXS pattern.

**Results**

The evolution of the hydrogel fiber volume during tensile loading was characterized during the loading cycles of Example 6, in the case of the dry-spun hydrogel fibers. For that, the diameter of the fiber was measured from images taken during loading cycles. The evolution of the diameter as a function of tensile strain is shown in **Figure 23A** for the $1^{st}$ and the $5^{th}$ cycles. For each cycle, the curve follows the same path during loading and unloading and the curves for both cycles superimpose perfectly. The data are well fitted by a linear law of the type: $d = d_0 \cdot (1 - v \cdot \varepsilon)$, where $d_0$ is the initial diameter, v is the Poisson coefficient and $\varepsilon$ is the strain. Best fits are obtained for values of $v = 0.50 \pm 0.01$. This result indicates that variations in the volume of the fiber during loading and unloading are negligible and the hydrogel fiber behaves as an incompressible solid.

The evolution of the semi-crystalline microstructure of individual dry-spun hydrogel fibers during loading cycles and rest was characterized using an in-situ tensile apparatus on a synchrotron WAXS set-up. **Figure 24** show the 2D WAXS patterns obtained in different states. Upon stretching, a narrowing of the 101 and $10\overline{1}$ peaks in the direction perpendicular to the fibers axis was observed as seen when comparing **Figure 24A** and B. This indicates an increase in the

macromolecular orientation in the direction of stretching. Upon unloading, the 101 and 101 peaks broaden again and the 2D WAXS pattern in the unstretched state (**Figure 24C**) is similar to the initial one. After a 1 h rest, no change in the 2D WAXS pattern was observed (**Figure 24D**).

The observations above are confirmed quantitatively by plotting intensity profiles as shown in **Figure 25**. In these plots, the reduction of the scattering volume caused by stretching the fiber needs to be corrected. Considering that the fiber is incompressible, this effect is corrected by multiplying the intensity by $(1+\varepsilon)$ where $\varepsilon$ is the tensile strain. The corrected intensity profiles as a function of the scattering angle $2\theta$ (**Figure 25A**) show the 101 and $10\overline{1}$ peaks centered on $2\theta = 15.7°$. The profiles for the initial swelling state, the unstretched state after 2 cycles and the recovered after 1 h rest superimpose perfectly. This indicates that the crystallinity of the fiber was not altered by the loading cycles and did not vary during rest. Similarly, the corrected intensity profiles as a function of the azimuthal angle $\psi$ (**Figure 25B**) show a preferential orientation in the direction of the fiber. The perfect superimposition of the profiles for the initial swelling state, the unstretched state after 2 cycles and the recovered after 1 h rest demonstrates that the orientation at the macromolecular scale was not altered by the loading cycles and did not vary during rest.

These results provide insightful information about the origins of the hysteresis and of the recovery upon rest observed in the PVA hydrogel fibers. Incompressibility of the fiber indicates that the hysteresis observed during the 1$^{st}$ loading cycle is not attributable to the expulsion of water out of the hydrogel fiber and the changes in the mechanical response between the cycles are due to changes in the macromolecular assembly composing the fiber. In situ WAXS measurements showing the preservation of the crystalline microstructure during loading and unloading indicate that, for strains lower than the limit of elasticity (near 40% for the dry-spun hydrogel fiber), the hysteresis does not result from a destruction or disorientation of the PVA crystallites. Similarly, the absence of changes in the crystalline microstructure during rest indicates that the slow reformation and reorganization of crystallites are not responsible for the recovery.

Within the swollen semi-crystalline material, the swelling agent (water) cannot penetrate into the crystals and is therefore only located in the amorphous areas. As a result, the local PVA concentration in the amorphous areas is given by $\varphi_{\square\square\square} = 1 / [1 + (Q-1)/(1-Xc)]$ where Q is the swelling ratio and Xc is the crystallinity. The values of $\varphi_{PVA}$ are $0.25 \pm 0.10$ wt% for the dry-spun hydrogel fibers and $0.12 \pm 0.03$ wt% for the wet-spun hydrogel fibers. At such high polymer concentrations, PVA chains are expected to associate through hydrogen-bonds (Kawanishi et al. "Thermodynamic consideration of the sol-gel transition in polymer solutions" Polymer vol. 28 (1987): 980-84). The application of tensile strain can therefore lead to the dissociation of weak bonds between PVA segments within the amorphous regions. During rest, the reformation of these weak bonds is governed by the local dynamics of the polymer segments in the concentrated amorphous regions which is much slower than the typical strain rates applied during loading-unloading cycles. This molecular picture explains both the hysteresis produced during the first loading-unloading cycle and the progressive recovery of the mechanical properties upon rest over characteristic times of the order of 1.

Example 8: Fatigue resistance of dry-spun PVA hydrogel fibers.

**Materials and Methods**

The dry-spun PVA fibers presented in Example 1 were used.

The fatigue behavior of the dry-spun PVA hydrogel fibers was characterized using a dynamical test bench (Electroforce, Bose) equipped with two motors (Electroforce 200N, Bose), a 2 N submersible load-cell and a thermally regulated chamber filled with water. All experiments were performed at 37°C. In each experiment, one single dry-spun PVA fiber was mounted on the testing apparatus and left to swell free of any tension for 30 min to reach equilibrium swelling. After this time, a small force of 0.003 N was applied to align the fiber.

The effect of self-recovery on the fatigue resistance was investigated by applying the protocol depicted in **Figure 26**. The following sequence was repeated: (i) a first triangular cycle (cycle A) from 0% to 40% tensile strain at 2.5 mm/s (about 20%/s); (ii) $10^4$ sinusoidal cycles between 10% and 40% at 5 Hz; (iii) a second triangular cycle (cycle B) from 0% to 40% tensile strain at 2.5 mm/s; (iv) a rest time at 0%. The sequence was repeated 12 times, which corresponds to a total of 120,000 sinusoidal cycles, referred to as "fatigue cycles".

Several values of rest times were tested: 0 min (no rest), 30 min, 8 h. Experiments were repeated on four samples for the no rest time and two samples for the 30 min and 8 h rest times.

Residual strain $\varepsilon_{Res}$ was measured for all the cycles A and B as the lowest strain for which the force increased above 0.005 N during the loading phase of each cycle.

Young's modulus E was measured for all the cycles A and B by taking the slope of a linear fit of the loading stress-strain curves between a strain of 10% and 20%.

The force $F_{35}$ reached during loading at a strain of 35% was measured for all the cycles A and B.

The hysteresis U was measured for all the cycles A and B by measuring the area between the loading and the unloading stress-strain curves of each cycle.

Residual strain, force at 35% strain upon loading, Young's modulus and hysteresis were measured for all the

triangular cycles A and B using the methods described in Example 5. Those data were normalized by the values obtained after the first $10^4$ sinusoidal cycles.

**Results**

The evolution of the mechanical properties of dry-spun hydrogel fibers during fatigue was monitored through the analysis of the triangular cycles A and B. These results are show in **Figure 27** (cycles A) and **Figure 28** (cycles B) for different rest times (no rest, 30 min, 8 h).

In the absence of rest (full circle symbols), the fatigue loading produced a continuous alteration of the mechanical response of the fibers. The residual strain continuously increased with the number of fatigue cycles with a 50% increase between $10^4$ and $10^5$ cycles (**Figures 27A** and **28A**). The Young's modulus (**Figures 27B** and **28B**), the force at 35% strain (**Figures 27C** and **28C**) and the hysteresis (**Figures 27D** and **28D**) continuously decreased with the number of fatigue cycles.

For a 30 min rest time (upward triangle symbols), a reduced alteration of the mechanical response was observed just after the rest periods (cycles A). For instance, the increase in residual strain (**Figure 27A**) and the decrease in Young's modulus (**Figure 27 B**) were significantly less pronounced after the 30 min rest than in the absence of rest. Nevertheless, this beneficial effect of the 30 min rest disappeared progressively for large numbers of cycles. Moreover, just after the application of each series of $10^4$ cycles (cycles B), no difference in mechanical response was observed between the no rest and the 30 min rest experiments, as shown in **Figure 28**. This indicates that the benefits of the 30 min rest vanished during each series of $10^4$ cycles.

For an 8h rest time (downward triangle symbols), the benefits of rest were very substantial and robust. Just after the rest periods (cycles A), the mechanical response was significantly superior to the response obtained without any rest (**Figure 27**). The residual strain and the Young's modulus remained fairly constant from $10^4$ to $10^5$ fatigue cycles. This fatigue resistance induced by rest is clearly illustrated in **Figure 29** showing the perfect superposition of the stress-strain curves of cycles A. Remarkably, the benefits of the 8h rest were persistent and remained effective even after each series of $10^4$ fatigue cycles, as shown in **Figure 28**. In particular, steady values of residual strain (**Figure 28A**) and Young's modulus (**Figure 28B**) were measured without any noticeable effect of the fatigue loadings.

Interestingly, the reported fatigue resistance induced by rest is efficient for applied loading histories that are representative of the loading of most human unmineralized connective tissues and in particular tissues like ligaments, tendons and menisci ($10^4$ flexion-extension cycles of the joints per day, 8h daily rest).

**Claims**

**1.** A swollen fiber assembly comprising at least two swollen fibers, each swollen fiber comprising a polymer and at least one swelling agent, the polymer being swollen with the least one swelling agent and consisting of at least two polymer chains, the at least two polymer chains being:

- semi-crystalline,
- linked together by weak bonds in amorphous areas, and
- oriented on a macromolecular scale in the swollen state,

the at least two fibers being non-woven, twisted, woven, braided or knitted together, or included within a polymeric matrix further comprised in the swollen fiber assembly.

**2.** The swollen fiber assembly according to claim **1**, wherein the at least two polymer chains are linked together by hydrogen bonds and/or the amorphous areas are located between crystallites in the direction of the length of each chain of the at least one polymer.

**3.** The swollen fiber assembly according to claim **1** or **2**, wherein the crystallinity of each fiber is greater than 15%, preferably it ranges from 20% to 80%, more preferably it ranges from 25% to 70%, even more preferably it ranges from 30% to 65%, the crystallinity being measured by differential scanning calorimetry on the fibers in the dry state.

**4.** The swollen fiber assembly according to any one of claims **1** to **3**, wherein the weight of the polymer of each swollen fiber represents from 5% to 90% of the total weight of said swollen fiber at room temperature, preferably the weight of the polymer of each swollen fiber represents from 20% to 80% of the total weight of said swollen fiber at room temperature, more preferably the weight of the polymer of each swollen fiber represents from 30% to 70% of the total weight of said swollen fiber at room temperature, even more preferably the weight of the polymer of each swollen fiber

represents about 50% of the total weight of said swollen fiber at room temperature.

5. The swollen fiber assembly according to any one of claims **1** to **4**, wherein the polymer is selected from the group consisting of: poly(vinyl alcohol)s, poly(urethane)s, poly(ester)s, poly(saccharide)s, proteins, synthetic polypeptides, and any copolymers thereof, preferably the polymer consists of poly(vinyl alcohol).

6. The swollen fiber assembly according to any one of claims **1** to **5**, wherein the at least two polymer chains are poly(vinyl alcohol) chains, preferably chains of poly(vinyl alcohol) having an average molar mass ranging from 5,000 g/mol to 400,000 g/mol, more preferably from 25,000 g/mol to 300,000 g/mol, even more preferably from 80,000 g/mol to 200,000 g/mol.

7. The swollen fiber assembly according to any one of claims **1** to **6**, wherein the linear density of each of the at least two swollen fibers is greater than 0.1 Dtex, preferably greater than 1 Dtex, more preferably greater than 10 Dtex, even more preferably greater than 100 Dtex.

8. The swollen fiber assembly according to any one of claims **1** to **7**, wherein the diameter of each swollen fiber ranges from 1 $\mu$m to 1000 $\mu$m, preferably from 5 $\mu$m to 500 $\mu$m, more preferably from 10 $\mu$m to 200 $\mu$m, even more preferably from 15 $\mu$m to 150 $\mu$m.

9. The swollen fiber assembly according to any one of claims **1** to **8**, wherein the at least one swelling agent is selected from the group consisting of water, dimethyl sulfoxide, dimethylformamide and mixtures thereof, preferably the at least one swelling agent is water.

10. The swollen fiber assembly according to any one of claims **1** to **9**, wherein the weight of the at least one swelling agent represents at least 10% of the total weight of each swollen fiber at room temperature, preferably the weight of the at least one swelling agent represents from 20% to 80% of the total weight of each swollen fiber at room temperature, more preferably the weight of the at least one swelling agent represents from 30% to 70% of the total weight of each swollen fiber at room temperature, even more preferably the weight of the at least one swelling agent represents about 50% of the total weight of each swollen fiber at room temperature.

11. A method for obtaining a self-recovery phenomenon of a swollen fiber assembly according to any one of claims **1** to **10**, comprising the following steps:

    - applying a constant mechanical stress or a loading-unloading cycle or a series of loading-unloading cycles on said swollen fiber assembly, then
    - applying a resting time of at least 30 minutes, preferably of at least 1 hour, more preferably of at least 2 hours, even more preferably of about 8 hours, on the swollen fiber assembly.

12. Use of a swollen fiber assembly according to any one of claims **1** to **10** as or in an implant, as or in an orthosis, as or in exoskeleton, or in a robotic structure.

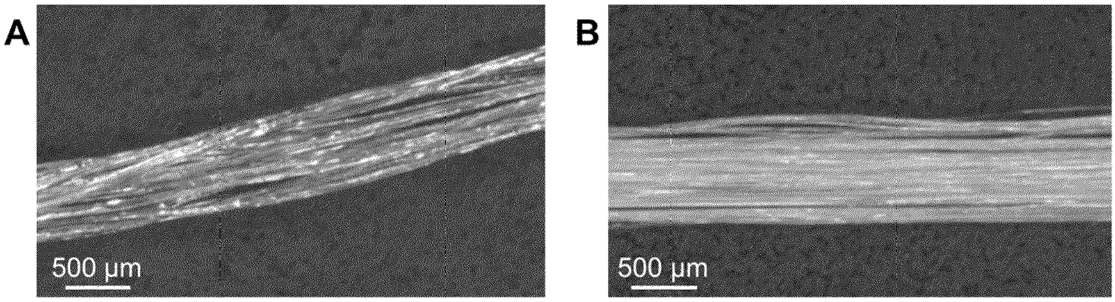


FIG. 1

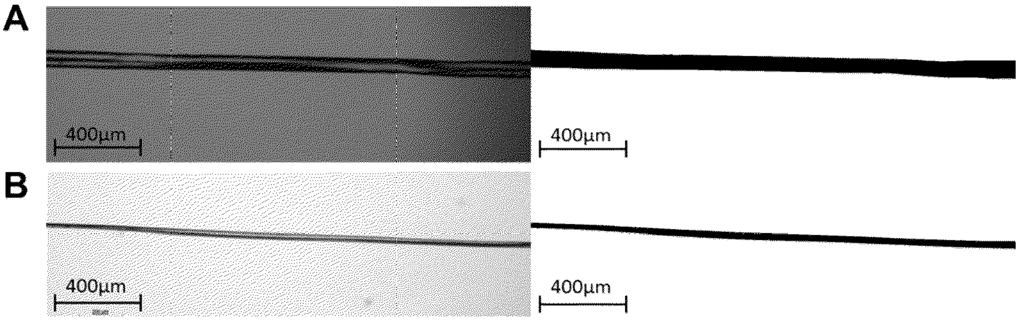


FIG. 2

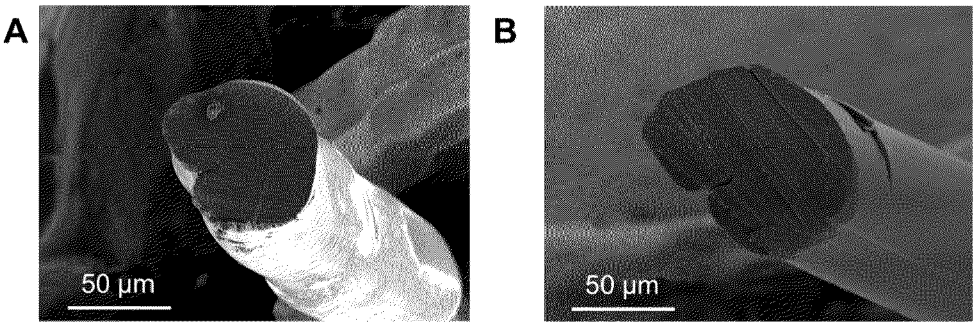


FIG. 3

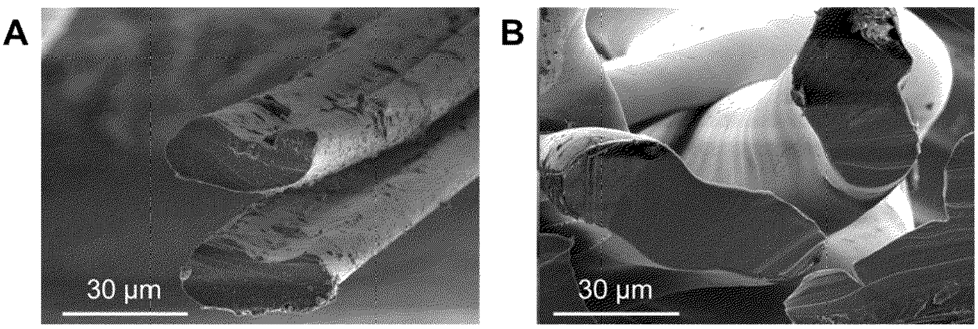


FIG. 4

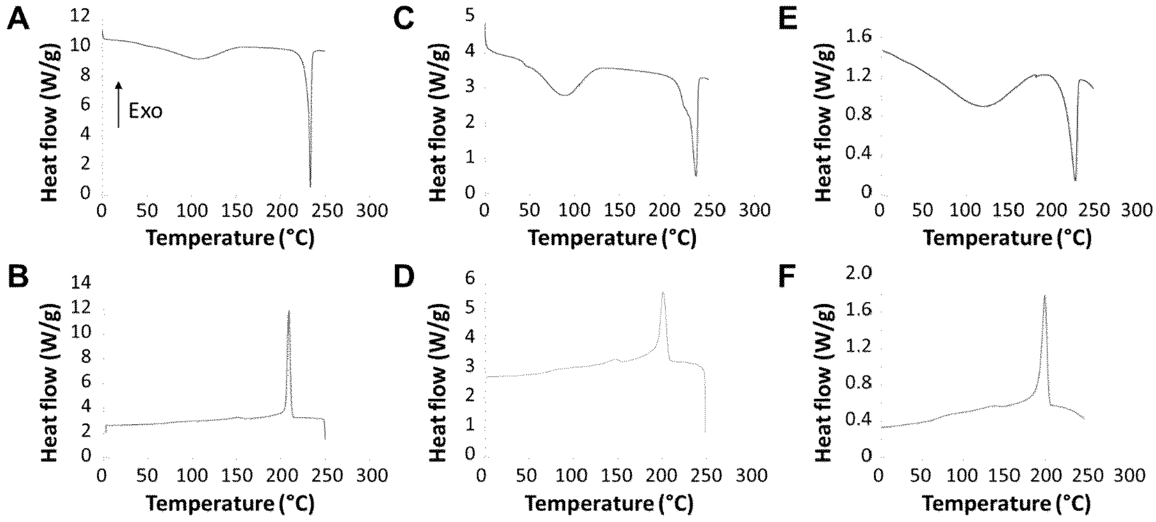


FIG. 5

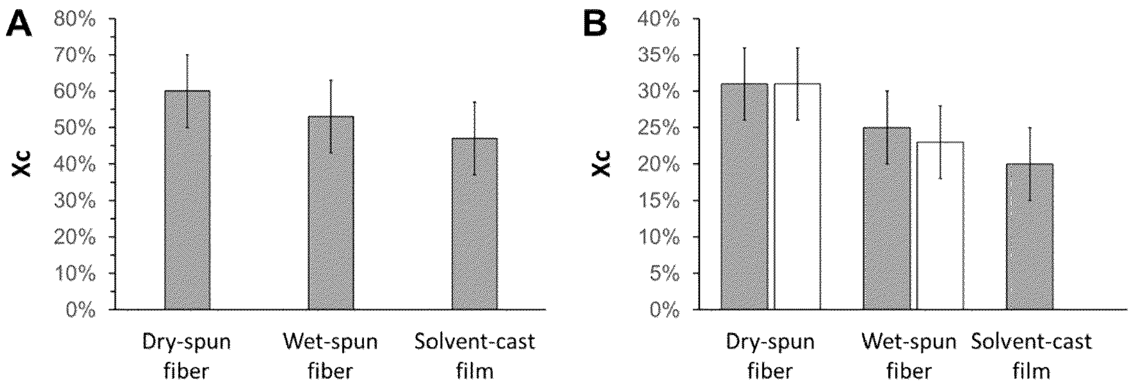


FIG. 6

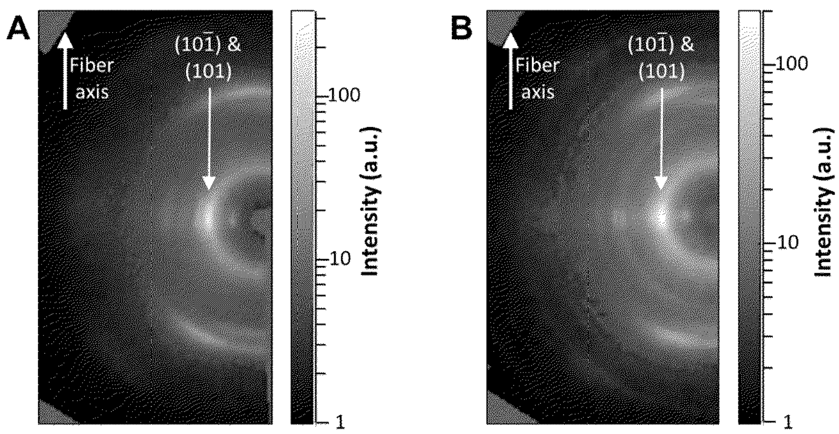


FIG. 7

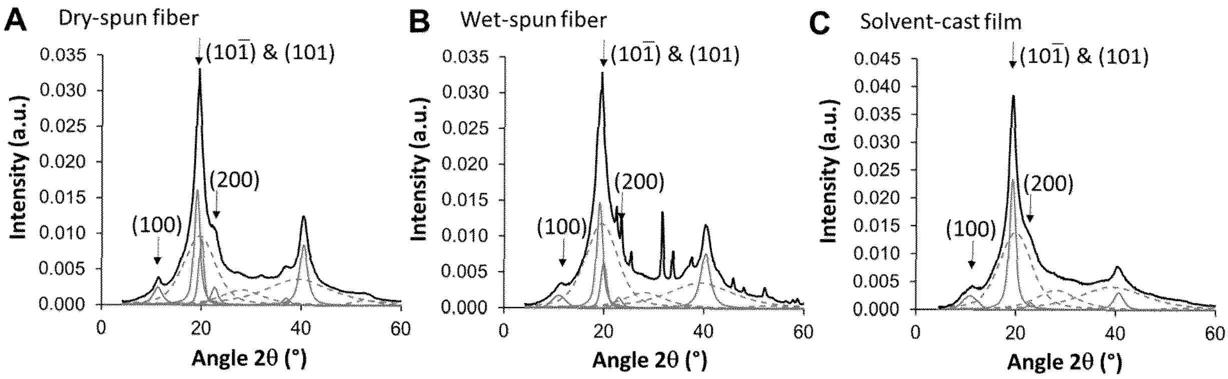


FIG. 8

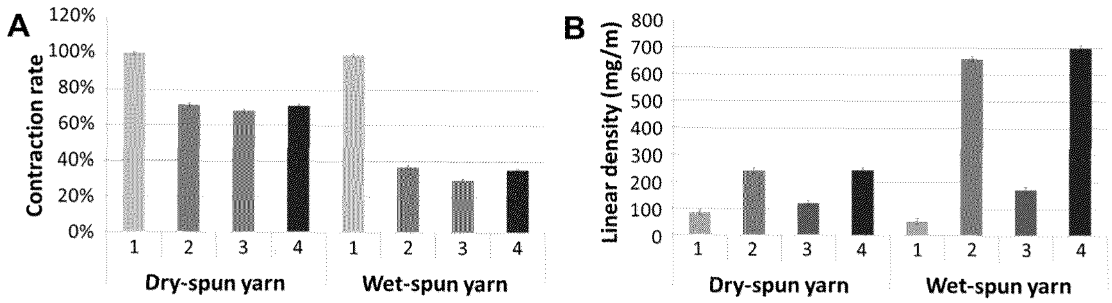


FIG. 9

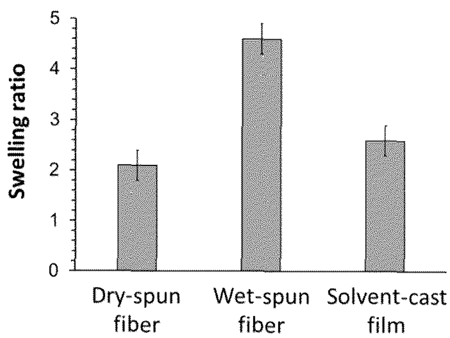


FIG. 10

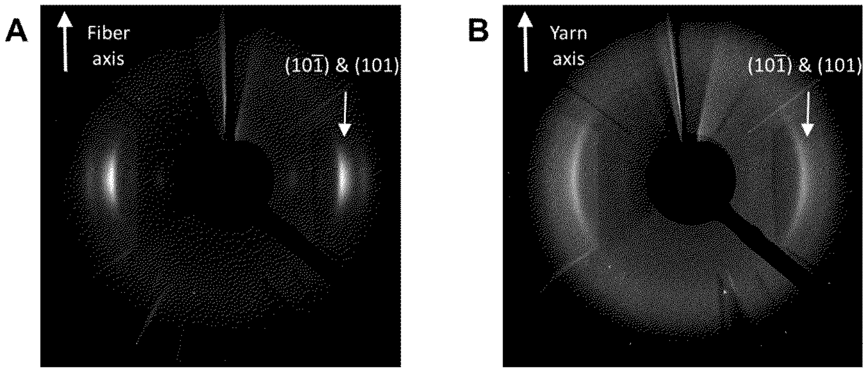


FIG. 11

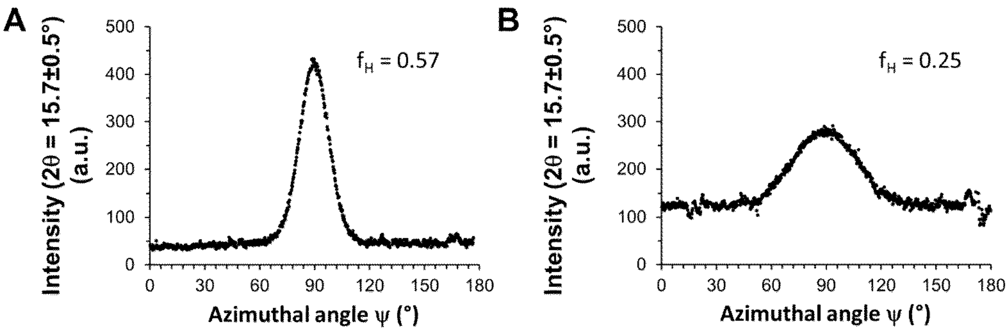


FIG. 12

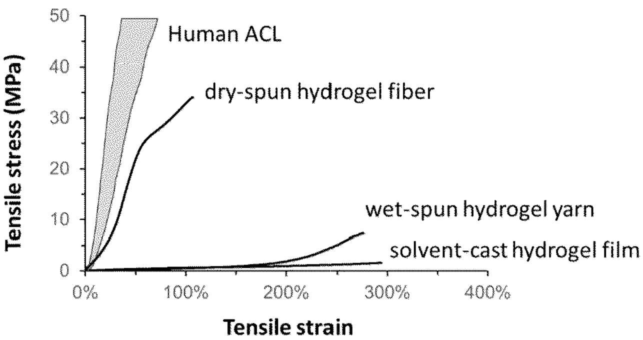


FIG. 13

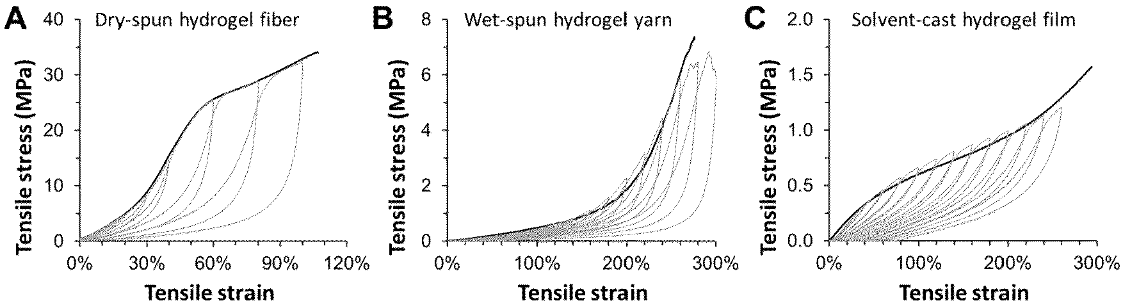


FIG. 14

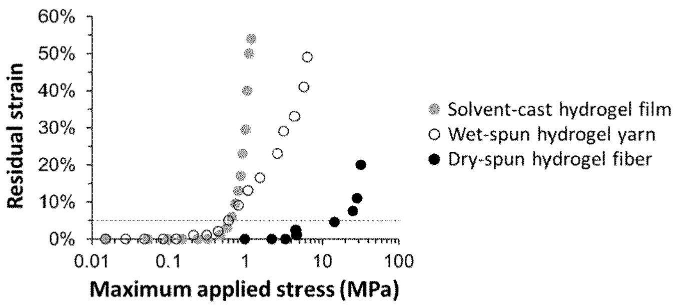


FIG. 15

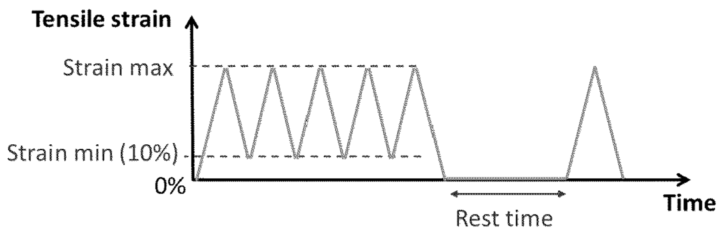


FIG. 16

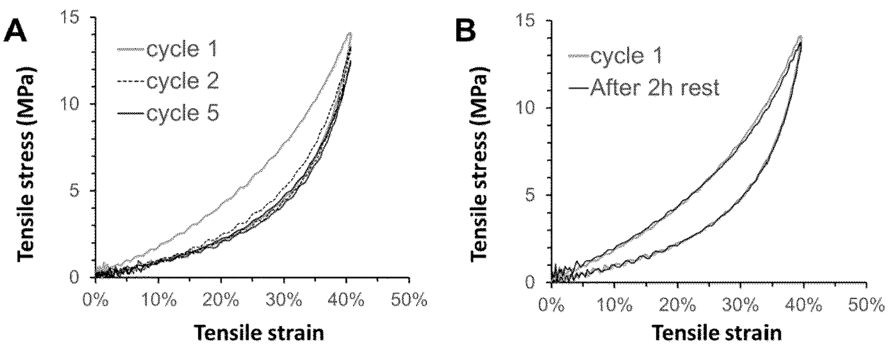


FIG. 17

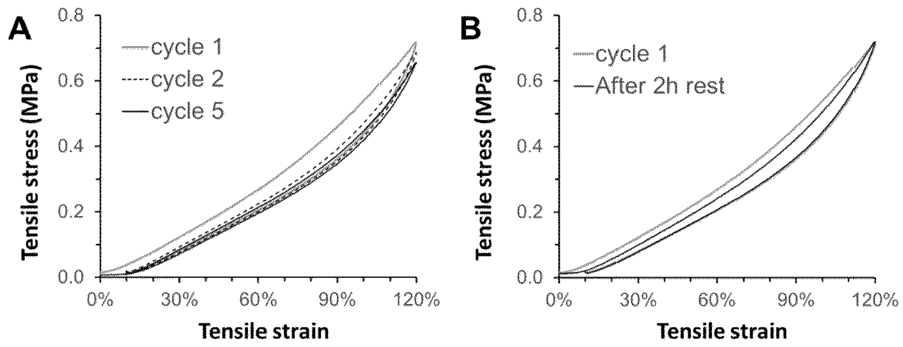


FIG. 18

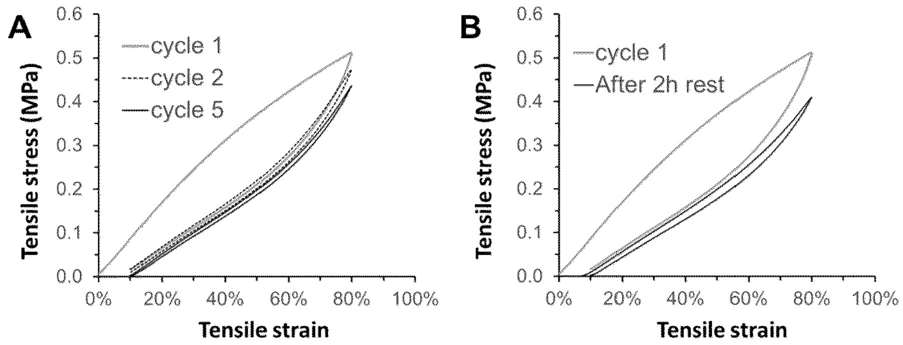


FIG. 19

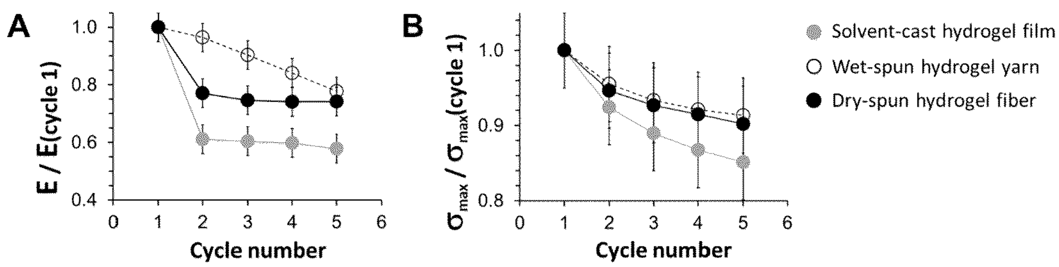


FIG. 20

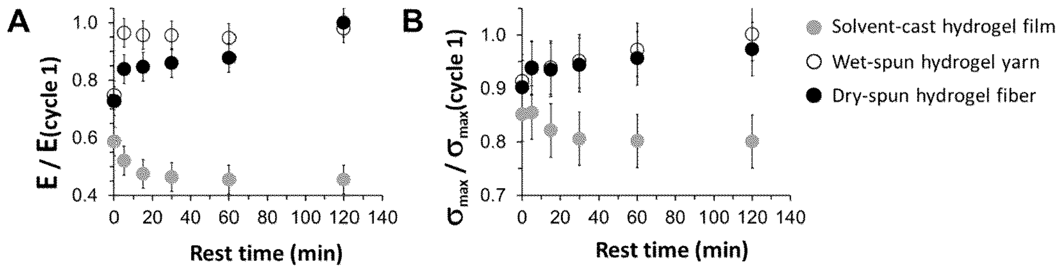


FIG. 21

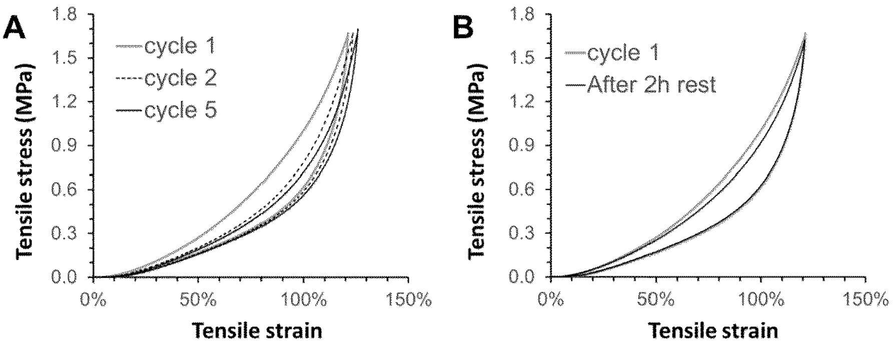


FIG. 22

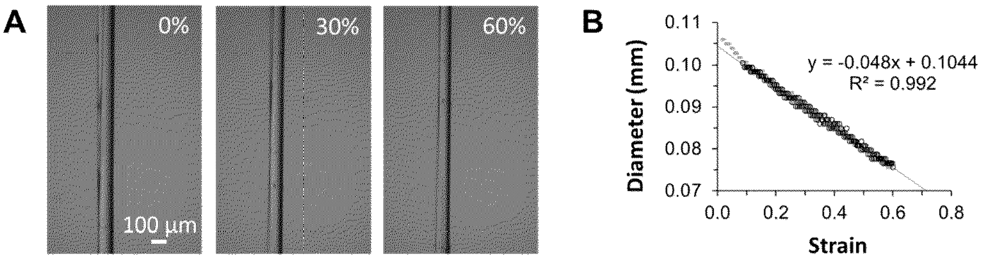


FIG. 23

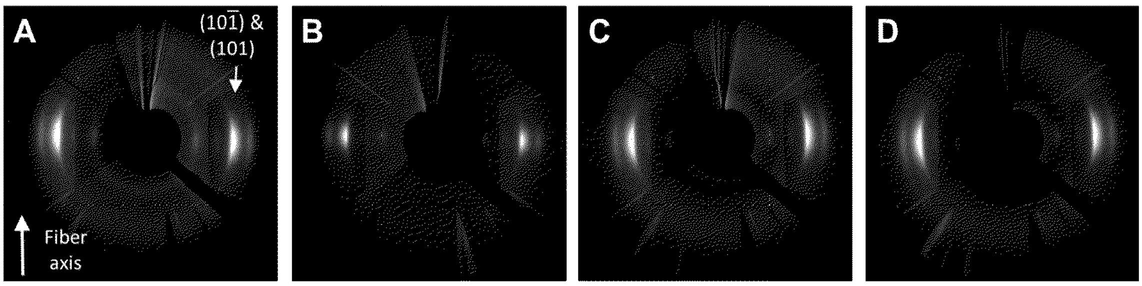


FIG. 24

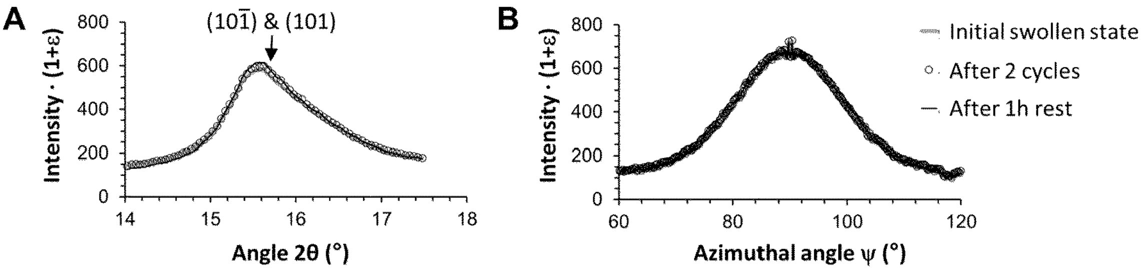


FIG. 25

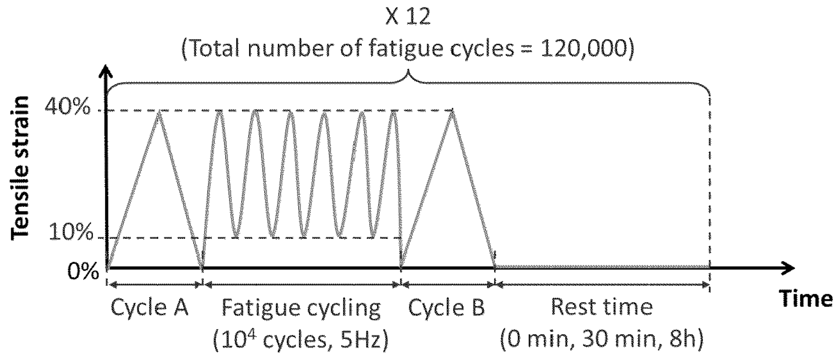


FIG. 26

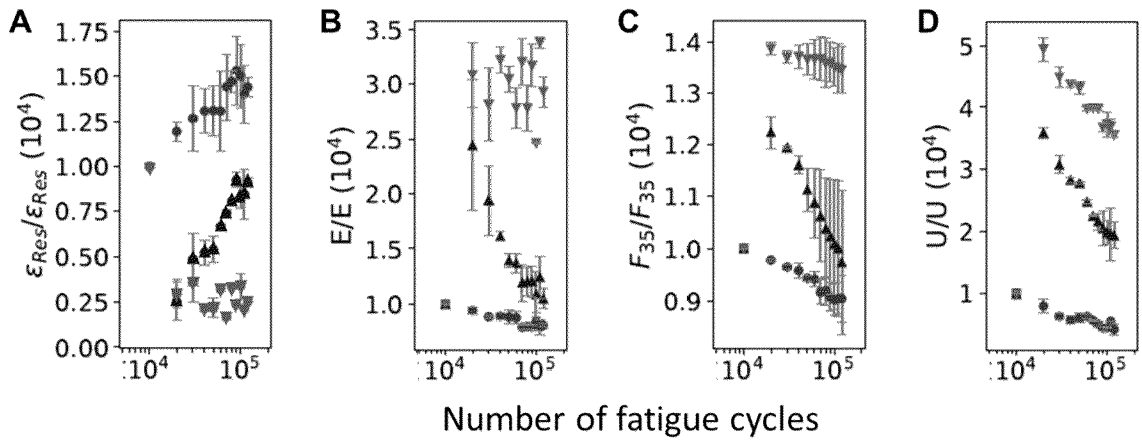


FIG. 27

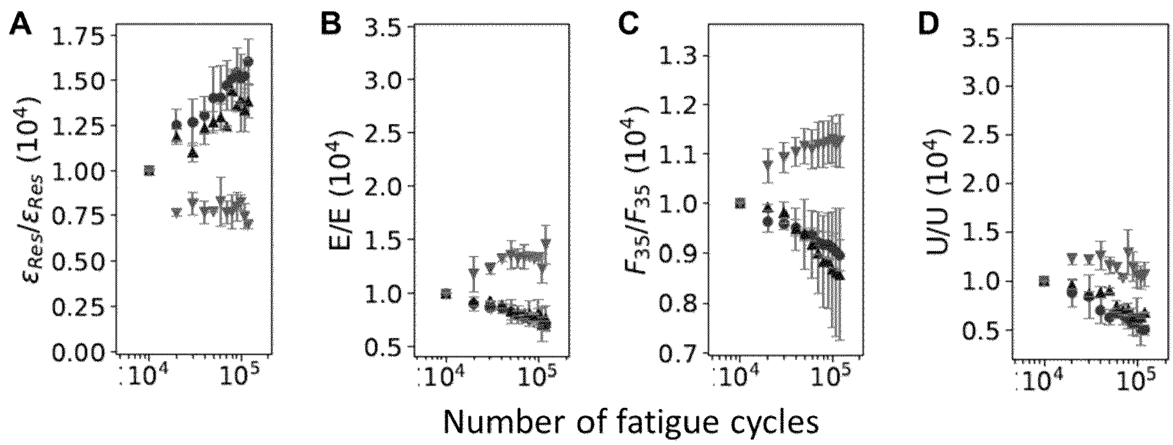


FIG. 28

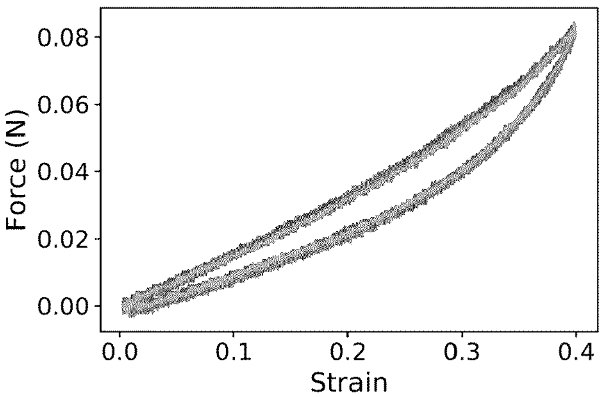


FIG. 29

Europäisches Patentamt
European Patent Office
Office européen des brevets

# EUROPEAN SEARCH REPORT

Application Number
EP 23 30 5984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/274415 A1 (SCHMITZ WIEBKE [DE] ET AL) 17 October 2013 (2013-10-17)<br>* paragraphs [0003], [0014], [0020], [0023], [0024], [0029], [0032], [0043], [0045], [0088] *<br>----- | 1-10,12 | INV.<br>D06M11/05<br>D01F1/10<br>D01F6/14<br>D01F6/50<br>A61L27/52 |
| | | | TECHNICAL FIELDS SEARCHED (IPC) |
| | | | D06M<br>D01F<br>A61L |

The present search report has been drawn up for all claims

1

| Place of search | Date of completion of the search | Examiner |
|---|---|---|
| The Hague | 14 December 2023 | Blas, Valérie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 30 5984

This annex lists the patent family members relating to the patent documents cited in the above-mentioned European search report. The members are as contained in the European Patent Office EDP file on
The European Patent Office is in no way liable for these particulars which are merely given for the purpose of information.

14-12-2023

| Patent document cited in search report | Publication date | Patent family member(s) | Publication date |
|---|---|---|---|
| US 2013274415 A1 | 17-10-2013 | CN 103374766 A | 30-10-2013 |
| | | DE 102012007307 A1 | 17-10-2013 |
| | | DK 2650413 T3 | 29-07-2019 |
| | | EP 2650413 A1 | 16-10-2013 |
| | | ES 2732070 T3 | 20-11-2019 |
| | | PL 2650413 T3 | 29-11-2019 |
| | | TR 201909187 T4 | 22-07-2019 |
| | | US 2013274415 A1 | 17-10-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KOLBUK et al.** Optical birefringence and molecular orientation of electrospun polycaprolactone fibers by polarizing-interference microscopy. *European Polymer Journal*, 2012, vol. 48, 275-83
- **LAFRANCE, C P et al.** Study of the distribution of molecular orientation in highly oriented polyethylene by x-ray diffraction. *Macromolecules*, 1991, vol. 24, 4948-56
- **MOSELEY W W**. The measurement of molecular orientation in fibers by acoustic methods. *Journal of applied polymer science*, 1960, vol. 3, 266-76
- **BOULET-AUDET M et al.** Attenuated total reflection infrared spectroscopy: an efficient technique to quantitatively determine the orientation and conformation of proteins in single silk fibers. *Applied spectroscopy*, 2008, vol. 62, 956-62
- **ROUSSEAU M-E et al.** Study of protein conformation and orientation in silkworm and spider silk fibers using Raman microspectroscopy. *Biomacromolecules*, 2004, vol. 5, 2247-57
- **PEPPAS, N. A. et al.** Crystallization kinetics of poly(vinyl alcohol). *Journal of applied polymer science*, 1982, vol. 27, 4787-4797
- **WOO et al.** Tensile properties of the human femur-anterior cruciate ligament-tibia complex The effects of specimen age and orientation. *Am. J. Sports Med.*, 1991, vol. 19 (3), 217-225
- **KAWANISHI et al.** Thermodynamic consideration of the sol-gel transition in polymer solutions. *Polymer*, 1987, vol. 28, 980-84